# EUROPEAN PATENT SPECIFICATION

(11) **EP 4 284 410 B1**
(45) Date of publication and mention of the grant of the patent: **02.07.2025**
(21) Application number: 22705498.8
(22) Date of filing: 28.01.2022
(51) Int. Cl.: A61K 38/26, A61K 35/17, A61P 37/02

(54) **APRAGLUTIDE FOR USE IN THE TREATMENT OF GASTROINTESTINAL GRAFT VERSUS HOST DISEASE**
APRAGLUTID ZUR VERWENDUNG BEI DER BEHANDLUNG DER GASTROINTESTINALEN GAFT-VERSUS-HOST-KRANKHEIT
APRAGLUTIDE POUR UTILISATION DANS LE TRAITEMENT DE LA MALADIE GASTRO-INTESTINALE DU GREFFON CONTRE L'HÔTE

(30) Priority: 28.01.2021 US 202163142905 P; 24.09.2021 US 202163248074 P
(43) Date of publication of application: 06.12.2023
(73) Proprietor: VectivBio AG, 4051 Basel (CH)
(72) Inventor: DIMITRIADOU, Violetta, 4051 Basel (CH); YOUSSEF, Nader N., 4051 Basel (CH)
(74) Representative: Cooley (UK) LLP
(86) International application number: PCT/US2022/014349
(87) International publication number: WO 2022/165204

(56) References cited:
- US-A1- 2020 254 065
- SLIM GEORGE M. ET AL: "Novel Long‐Acting GLP‐2 Analogue, FE 203799 (Apraglutide), Enhances Adaptation and Linear Intestinal Growth in a Neonatal Piglet Model of Short Bowel Syndrome with Total Resection of the Ileum", JPEN - JOURNAL OF PARENTERAL AND ENTERAL NUTRITION, vol. 43, no. 7, 6 January 2019 (2019-01-06), US, pages 891 - 898, XP055825588, ISSN: 0148-6071, Retrieved from the Internet <URL:https://onlinelibrary.wiley.com/doi/full-xml/10.1002/jpen.1500> DOI: 10.1002/jpen.1500
- HARGROVE DIANE M. ET AL: "Pharmacological Characterization of Apraglutide, a Novel Long-Acting Peptidic Glucagon-Like Peptide-2 Agonist, for the Treatment of Short Bowel Syndrome", JOURNAL OF PHARMACOLOGY AND EXPERIMENTAL THERAPEUTICS, vol. 373, no. 2, 13 April 2020 (2020-04-13), US, pages 193 - 203, XP055813396, ISSN: 0022-3565, DOI: 10.1124/jpet.119.262238
- NORONA JOHANA ET AL: "Glucagon-like peptide 2 for intestinal stem cell and Paneth cell repair during graft-versus-host disease in mice and humans", BLOOD, vol. 136, no. 12, 17 September 2020 (2020-09-17), US, pages 1442 - 1455, XP055825845, ISSN: 0006-4971, Retrieved from the Internet <URL:http://ashpublications.org/blood/article-pdf/136/12/1442/1758126/bloodbld2020005957.pdf> DOI: 10.1182/blood.2020005957

## Description

### BACKGROUND

Graft versus Host Disease (GvHD) is a condition that occurs following a transplant in a subject. GvHD is most commonly observed in the context of an allogeneic transplants, including hematopoietic stem cell transplantation (AHCT) for the treatment of a blood cancer. However, there is increasing evidence that GvHD also occurs within the context of autologous transplants (Hammami et al., Gastroenterol Res., 2018, 11(1):52-57; Cogbill et al. Modern Pathology, 2011, 24:117-125). GvHD occurs when immune cells present in the allogeneic transplant perceive the transplant recipient's tissues as foreign and proceed to attack the recipient's tissues. GvHD may manifest as either acute GvHD or chronic GvHD. Acute GvHD is typically characterized by inflammation and tissue damage in the skin, oral and genital mucosa, eyes, gut, liver, lungs, joints, and muscles. Chronic GvHD can induce the same type of damage, but over longer periods can also cause damage to connective tissue of exocrine glands, tissue fibrosis and limitation of joint motility, fibrosis of the lungs and liver, immune dysregulation and autoimmunity. In particular, acute GvHD of the gastrointestinal tract can result in severe intestinal inflammation, sloughing of the mucosal membrane, severe diarrhea, abdominal pain, nausea and vomiting. Severe manifestations of acute gastrointestinal GvHD are often seen in patients with poorer, post-transplant prognoses. First-line treatment of acute gastrointestinal GvHD typically comprises the use of systemic and/or oral non-absorbable corticosteroids. However, a large number of patients fail to respond to first-line therapy. Thus, there exists a need in the art for compositions and methods directed to the treatment and prevention of GvHD, more specifically acute gastrointestinal GvHD, including in subjects that have received an AHCT. This disclosure addresses that need.

SLIM, George M. et al disclose "Novel Long Acting GLP-2 Analogue, FE203799 (Apraglutide), Enhances Adaptation and Linear Intestinal Growth in a Neonatal Piglet Model of Short Bowel Syndrome with Total Resection of the Ileum" in JOURNAL OF PARENTERAL AND ENTERAL NUTRITION, (2019), 43(7), p. 891-898. HARGROVE, Diane M. et al disclose "Pharmacological Characterization of Apraglutide, a Novel Long-Acting Peptidic Glucagon-Like Peptide-2 Agonist, for the Treatment of Short Bowel Syndrome" in JOURNAL OF PHARMACOLOGY AND EXPERIMENTAL THERAPEUTICS, (2020), 373(2), p. 193-203. US 2020/0254065 A1 discloses long-acting GLP-2 analogs. NORONA, Johana et al disclose "Glucagon-like peptide 2 for intestinal stem cell and Paneth cell repair during graft-versus-host disease in mice and humans" in BLOOD, (2020), 136(12), p. 1442-1455.

### SUMMARY

The invention is defined by the appended claims. The present invention provides apraglutide, or a pharmaceutically acceptable salt thereof, for use in the treatment or prevention of acute gastrointestinal GvHD in a subject. The acute gastrointestinal GvHD can be steroid-refractory. The acute gastrointestinal GvHD can be steroid-naive. In some embodiments, the acute gastrointestinal GvHD is grade II- IV acute gastrointestinal GvHD according to the MAGIC scale.

In some aspects, the apraglutide or pharmaceutically acceptable salt thereof can be administered to the subject prior to the subject being administered a transplant.

In some aspects, the apraglutide or pharmaceutically acceptable salt thereof can be administered prior the subject being administered radiation therapy, chemotherapy, or radiomimetic therapy in connection with a transplant.

In some aspects, the subject can have been previously administered a transplant.

In some aspects, the apraglutide or pharmaceutically acceptable salt thereof can be administered to the subject after the subject has been administered a transplant.

In some aspects, the subject can have been previously administered radiation therapy, chemotherapy, radiomimetic therapy or any combination thereof in connection with a transplant.

In some aspects, the apraglutide or pharmaceutically acceptable salt thereof can be administered to the subject after the subject has been administered radiation therapy, chemotherapy, radiomimetic therapy or any combination thereof.

In some aspects, the apraglutide or pharmaceutically acceptable salt thereof can be administered to the subject after the subject has been administered radiation therapy, chemotherapy, radiomimetic therapy or any combination thereof and prior to the subject being administered a transplant.

In some aspects, the apraglutide or pharmaceutically acceptable salt thereof can be administered to the subject concurrently with a transplant.

In some aspects, the apraglutide or pharmaceutically acceptable salt thereof can be administered to the subject concurrently with a radiation therapy, a chemotherapy, a radiomimetic therapy, or any combination thereof in connection with a transplant.

In some aspects, the administration of apraglutide can prevent and/or attenuate a reduction in colon length in a subject following a transplant.

In some aspects, the administration of apraglutide can prevent and/or attenuate a reduction in colon length in a subject following a conditioning therapy and a transplant.

In some aspects, the transplant can comprise hematopoietic stem cells derived from bone marrow, peripheral blood, umbilical cord blood or any combination thereof. The transplant can comprise T-cells. The T-cells can be chimeric antigen receptor (CAR) T-cells.

In some aspects, the radiation therapy can comprise total body irradiation.

In some aspects, the pharmaceutically acceptable salt of apraglutide can be the sodium salt of apraglutide.

In some aspects, the apraglutide, or pharmaceutically acceptable salt thereof, can be administered by subcutaneous injection.

In some aspects, the apraglutide, or pharmaceutically acceptable salt thereof, can be administered in an amount of between about 1 mg to about 10 mg.

In some aspects, apraglutide can be administered in an amount of about 2.5 mg. In some aspects, apraglutide can be administered in an amount of about 5 mg. In some aspects, apraglutide can be administered in an amount of about 10 mg.

In some aspects, the subject has a body weight of less than about 50 kg and the apraglutide is administered in an amount of about 2.5 mg. In some aspects, the subject has a body weight of about 50 kg to about 60 kg and the apraglutide is administered in an amount of about 2.5 mg or about 5 mg. In some aspects, the subject has a body weight of about 60 kg to about 80 kg and the apraglutide is administered in an amount of about 4 mg or about 7.5 mg. In some aspects, the subject has a body weight of greater than about 80 kg and the apraglutide is administered in an amount of about 5 mg or about 10 mg.

In some aspects, the transplant can be an allogeneic transplant.

In some aspects, the transplant can be an autologous transplant.

In some aspects, the subject can have been previously administered at least one anti-GvHD therapy. In some aspects, the at least one anti-GvHD therapy can comprise steroid therapy.

Any of the above aspects, or any other aspect described herein, can be combined with any other aspect described herein.

Unless otherwise defined, all technical and scientific terms used herein have the same meaning as commonly understood by one of ordinary skill in the art to which this disclosure belongs. In the specification, the singular forms also include the plural unless the context clearly dictates otherwise; as examples, the terms "a," "an," and "the" are understood to be singular or plural and the term "or" is understood to be inclusive. By way of example, "an element" means one or more element. Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. Unless otherwise clear from the context, all numerical values provided herein are modified by the term "about." Unless specifically stated or obvious from context, as used herein, the term "or" is understood to be inclusive and covers both "or" and "and".

### BRIEF DESCRIPTION OF THE DRAWINGS

The above and further features will be more clearly appreciated from the following detailed description when taken in conjunction with the accompanying drawings.
FIG. 1 is a series of graphs showing colon length in an acute GvHD mouse model following treatment with apraglutide.
FIG. 2a-2b is a series of graphs showing the results of various histological analyses of the gut of an acute GvHD mouse model following treatment with apraglutide.

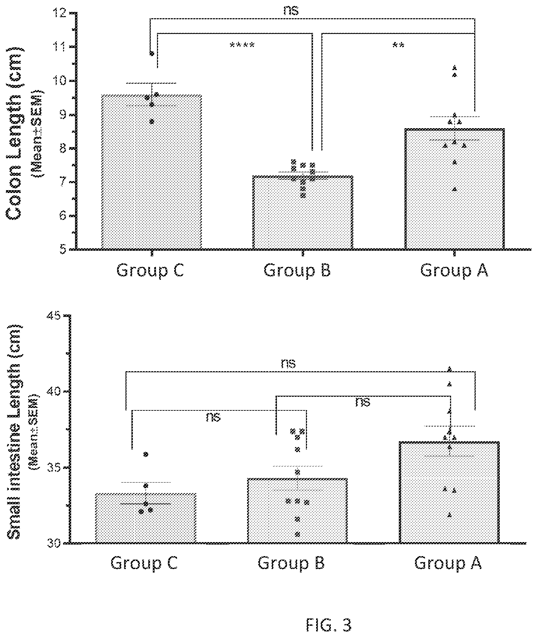

FIG. 3a-3b is a series of graphs showing colon length and small intestine length in an acute GvHD mouse model following treatment with apraglutide. Data are shown as the mean ± SEM length of intestinal colon. N-5-10/group. Each dot represents data for one mouse. Data show that the length of the colon of TBI/BMT animals is significantly shorter as compared to the vehicle group or the BMT/TBI group treated with apraglutide (Kruskal-Wallis non-parametric test, followed by the Dunn's test for multiple comparisons) (*p<0.05, ***<0.001). No difference was observed between the vehicle group and the TBI/BMT animals treated with apraglutide. BMT=bone marrow transplant; ns=not significant; SEM=standard error of the mean; TBI=total body irradiation.
FIG. 4 is a series of graphs showing the impact of apraglutide on engraftment of human PBMC in NOG irradiated mice.
FIG. 5 is a series of graphs showing that apraglutide protects BALB/cJ mice from acute GVHD-induced intestinal damage.
FIG. 6 is a series of graphs showing that apraglutide protects against chemotherapy-induced damage to small intestine.
FIG. 7 is a series of graphs showing that a combination of apraglutide with chemotherapy preserves the intestinal mass as indicated by plasma citrulline levels.
FIG. 8 is a series of graphs showing that apraglutide reduces chemotherapy-induced loss of body weight.
FIG. 9 is a series of graphs showing that a combination of apraglutide with chemotherapy improves animal survival.
FIG. 10a is a series of graphs showing that apraglutide maintains the composition of fecal microbiota during chemotherapy. F= Firmicutes and B= Bacteroidetes. Data are presented as % of relative abundance of taxa at the Phylum level in the stools/condition. FIG. 10b is a series of graphs showing that apraglutide stabilizes the diversity of the intestinal microbiota profoundly modified by chemotherapy (relative mean abundance of taxa in feces at the phylum level).
FIG. 11a is a series of graphs showing that apraglutide does not diminish antitumor efficacy of cytarabine.
FIG. 11b is a series of graphs showing the impact of vehicle or cytarabine ± apraglutide treatment on hCD45 positive cells in bone marrow and spleen in mice injected with human leukemia cells.
FIG. 12 is a series of graphs showing apraglutide's effect on the immunosuppression activity induced by Cytarabine (Mean/group ± SEM).
FIG. 13 is a series of graphs showing apraglutide's effect on the immunosuppression activity induced by Melphalan (Mean/group ± SEM).

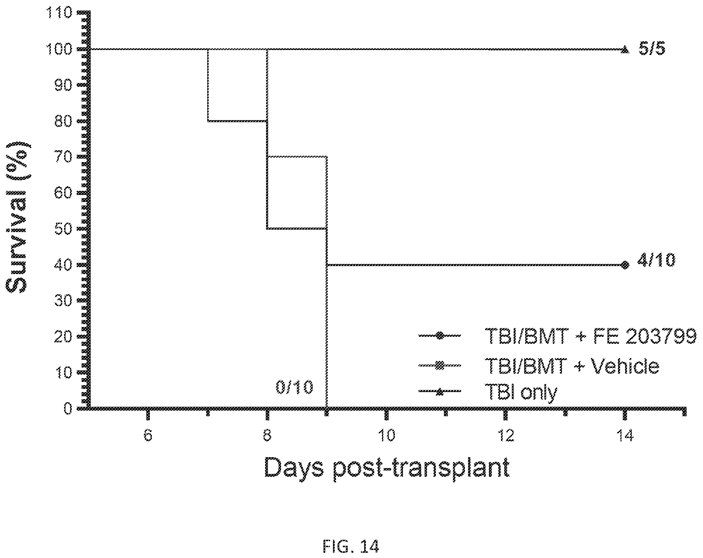

FIG. 14 is a series of graphs showing that treatment with apraglutide improves animal survival of TBI/BMT treated animals. Kaplan-Meier curves - Animal survival was monitored daily. N=10 at time=0 for each treatment group. Survival of animals treated with apraglutide initiated before TBI/BMT, showed significant difference relative to TBI/BMT animals as determined by log-rank (Mantel-Cox) test *p<0.03, but it was not significant (ns) as compared to the control animals. When comparing to the control group, survival of TBI/BMT was significant different ### p=0.0007. BMT=bone marrow transplant; TBI=total body irradiation.
FIG. 15a is a series of graphs showing that apraglutide improves overall pathological score following induction of cute GvHD. FIG. 15b is a series of graphs showing that apraglutide improves pathological intestinal score following induction of cute GvHD.
FIG. 16a-c is a series of graphs showing the effect of apraglutide on the percent weight loss in TBI/BMT treated animals.
FIG 17 is a series of graphs showing the dose-dependent effect of apraglutide on animal survival following cytarabine treatment.
FIG. 18 is a series of graphs showing the dose-dependent effect of apraglutide on animal body following chemotherapy.
FIG. 19 is a series of graphs showing the dose-dependent effect of apraglutide on serum citrulline concentration following treatment with cytarabine.
FIG. 20 is a series of graphs showing the dose-dependent effect of GP-2R agonists on polymorphonuclear cell count following chemotherapy.

### DETAILED DESCRIPTION

The present invention provides apraglutide, or a pharmaceutically acceptable salt thereof, for use in the treatment or prevention of acute gastrointestinal GvHD in a subject. In some embodiments, the apraglutide, or pharmaceutically acceptable salt thereof, is for administration to the subject in at least one therapeutically effective amount.

The present invention provides apraglutide, or a pharmaceutically acceptable salt thereof, for use in the treatment of acute gastrointestinal GvHD in a subject, wherein the apraglutide, or pharmaceutically acceptable salt thereof, is for administration to the subject in at least one therapeutically effective amount.

The present invention provides apraglutide, or a pharmaceutically acceptable salt thereof, for use in the treatment of acute gastrointestinal GvHD in a subject.

The present invention provides apraglutide, or a pharmaceutically acceptable salt thereof, for use in the prevention of acute gastrointestinal GvHD in a subject, wherein the apraglutide, or pharmaceutically acceptable salt thereof, is for administration to the subject in at least one therapeutically effective amount.

The present invention provides apraglutide, or a pharmaceutically acceptable salt thereof, for use in the prevention of acute gastrointestinal GvHD in a subject.

Also disclosed herein is the use of apraglutide, or a pharmaceutically acceptable salt thereof, in the manufacture of a medicament for treating or preventing GvHD in a subject, wherein the apraglutide, or pharmaceutically acceptable salt thereof, is for administration to the subject in at least one therapeutically effective amount.

Also disclosed herein is the use of apraglutide, or a pharmaceutically acceptable salt thereof, in the manufacture of a medicament for treating or preventing GvHD in a subject.

Also disclosed herein is the use of apraglutide, or a pharmaceutically acceptable salt thereof, in the manufacture of a medicament for treating GvHD in a subject, wherein the apraglutide, or pharmaceutically acceptable salt thereof, is for administration to the subject in at least one therapeutically effective amount.

Also disclosed herein is the use of apraglutide, or a pharmaceutically acceptable salt thereof, in the manufacture of a medicament for treating GvHD in a subject.

Also disclosed herein is the use of apraglutide, or a pharmaceutically acceptable salt thereof, in the manufacture of a medicament for preventing GvHD in a subject, wherein the apraglutide, or pharmaceutically acceptable salt thereof, is for administration to the subject in at least one therapeutically effective amount.

Also disclosed herein is the use of apraglutide, or a pharmaceutically acceptable salt thereof, in the manufacture of a medicament for preventing GvHD in a subject.

Also disclosed herein is a method of treating or preventing GvHD in a subject, the method comprising administering to the subject at least one therapeutically effective amount of apraglutide, or a pharmaceutically acceptable salt thereof.

Also disclosed herein is a method of treating GvHD in a subject, the method comprising administering to the subject at least one therapeutically effective amount of apraglutide, or a pharmaceutically acceptable salt thereof.

Also disclosed herein is a method of preventing GvHD in a subject, the method comprising administering to the subject at least one therapeutically effective amount of apraglutide, or a pharmaceutically acceptable salt thereof.

Also disclosed herein is a method of treating or preventing GvHD in a subject, the method comprising administering to the subject apraglutide, or a pharmaceutically acceptable salt thereof.

Also disclosed herein is a method of treating GvHD in a subject, the method comprising administering to the subject apraglutide, or a pharmaceutically acceptable salt thereof.

Also disclosed herein is a method of preventing GvHD in a subject, the method comprising administering to the subject apraglutide, or a pharmaceutically acceptable salt thereof.

Also provided are suitable therapeutically effective amounts in which apraglutide, or a pharmaceutically acceptable salt thereof, can be used or administered to a subject for treating or preventing GvHD.

Also disclosed herein is a method of treating GvHD-induced intestinal damage in a subject who has been previously diagnosed with GvHD, the method comprising administering to the subject at least one therapeutically effective amount of apraglutide, or a pharmaceutically acceptable salt thereof. Also disclosed herein is a method of treating GvHD-induced intestinal damage in a subject who has been previously diagnosed with GvHD, the method comprising administering to the subject apraglutide, or a pharmaceutically acceptable salt thereof. The subject may have been previously diagnosed with GvHD and the GvHD is ongoing at the time of the administration of apraglutide. The subject may have been previously diagnosed with GvHD and the GvHD has been successfully treated prior to the administration of apraglutide.

In some aspects, the subject is older than 65 years. In some aspects, the subject is between 18 and 64 years old. In some aspects, the subject is younger than 18 years old.

In some aspects of, the subject weighs more than 50 kg. In some aspects, the subject weighs between 50 kg and 40 kg. In some aspects, the subject weighs less than 40 kg. The present disclosure provides apraglutide, or a pharmaceutically acceptable salt thereof, for use in treating GvHD-induced intestinal damage in a subject, wherein the apraglutide, or pharmaceutically acceptable salt thereof, is for administration to the subject in at least one therapeutically effective amount. The present disclosure provides apraglutide, or a pharmaceutically acceptable salt thereof, for use in treating GvHD-induced intestinal damage in a subject. In some aspects, the subject has been previously diagnosed with GvHD and the GvHD is ongoing at the time of the administration of apraglutide. In some aspects, the subject has been previously diagnosed with GvHD and the GvHD has been successfully treated prior to the administration of apraglutide.

Also disclosed herein is the use of apraglutide, or a pharmaceutically acceptable salt thereof, in the manufacture of a medicament for treating GvHD-induced intestinal damage in a subject, wherein the apraglutide, or pharmaceutically acceptable salt thereof, is for administration to the subject in at least one therapeutically effective amount. Also disclosed herein is the use of apraglutide, or a pharmaceutically acceptable salt thereof, in the manufacture of a medicament for treating GvHD-induced intestinal damage in a subject. The subject may have been previously diagnosed with GvHD and the GvHD is ongoing at the time of the administration of apraglutide. The subject ma have been previously diagnosed with GvHD and the GvHD has been successfully treated prior to the administration of apraglutide.

In some aspects, the GvHD-induced intestinal damage can include, but is not limited to malabsorption, diarrhea, abdominal pain, mucositis, mucosal ulceration, nausea, shortening of the colon, shortening of the small intestine and any other GvHD-induced gastrointestinal complication known in the art.

In accordance with the present invention, the GvHD is acute gastrointestinal GvHD.

In some aspects, the GvHD can be steroid-refractory acute gastrointestinal GvHD. In some aspects, the GvHD can be steroid-naive acute gastrointestinal GvHD.

Also disclosed herein is apraglutide, or a pharmaceutically acceptable salt thereof, for use in the treatment or prevention of chronic GvHD in a subject.

Chronic GvHD can be chronic gastrointestinal GvHD. The GvHD can be steroid-refractory chronic gastrointestinal GvHD, or may be steroid-naive chronic gastrointestinal GvHD.

The chronic GvHD may be late chronic GvHD. The chronic GvHD may be steroid-naive chronic GvHD. Alternatively, the chronic GvHD may be steroid-refractory chronic GvHD. The chronic GvHD may be chronic gastrointestinal GvHD, such as steroid-refractory chronic gastrointestinal GvHD. For example, apraglutide, or a pharmaceutically acceptable salt thereof, may be for use in the prevention of chronic GvHD, such as gastrointestinal GvHD, in a subject. For example, apraglutide, or a pharmaceutically acceptable salt thereof, may be for use in the treatment of steroid-refractory chronic GvHD, such as steroid-refractory chronic gastrointestinal GvHD, in a subject.

In the present disclosure, the GvHD can be Overlap Syndrome.

In some aspects, the subject has grade II- IV GvHD according to the MAGIC scale. In some aspects, the subject has grade III- IV GvHD according to the MAGIC scale. In some aspects, the subject has grade II- III GvHD according to the MAGIC scale. In some aspects, the subject has grade II, III or IV GvHD according to the MAGIC scale.

In some aspects, the GvHD is upper gut Graft versus Host Disease.

In some aspects, the GvHD is mid-lower gut Graft versus Host Disease.

Also disclosed herein is apraglutide, or a pharmaceutically acceptable salt thereof, for use in the prevention or treatment of liver GvHD in a subject.

The subject can be diagnosed with a cancer. The cancer may be a hematological cancer. The cancer can be at least one of Acute myeloid leukemia, myelodysplastic syndrome, follicular lymphoma, diffuse large B cell lymphoma, acute lymphoblastic leukemia, multiple myeloma, Hodgkin lymphoma, chronic myeloid leukemia, T cell non-Hodgkin lymphoma, lymphoblastic B cell non-Hodgkin lymphoma (non-Burkitt), Burkitt's lymphoma, anaplastic large cell lymphoma, germ cell tumor, Ewing's sarcoma, soft tissue sarcoma, neuroblastoma, Wilms' tumor, osteosarcoma, medulloblastoma, acute promyelocytic leukemia, mantle cell lymphoma, T cell lymphoma, lymphoplasmacytic lymphoma, cutaneous T cell lymphoma, plasmablastic lymphoma, chronic lymphocytic leukemia, breast cancer and renal cancer. For example, the subject can be diagnosed with a cancer and be designated to undergo an allogeneic transplant. Alternatively, the subject can be diagnosed with a cancer and has previously undergone an autologous transplant. Alternatively, the subject can be diagnosed with a cancer and has previously undergone an allogeneic transplant. Alternatively, the subject can be diagnosed with a cancer and has previously undergone an autologous transplant. The transplant may be in a subject having, for example, myelofibrosis. The transplant may be an autologous transplant in a subject having multiple myeloma.

The subject can be diagnosed with a disease or disorder. The disease or disorder may be at least one of a hemoglobinopathy, a congenital hemoglobinopathy, β-Thalessemia major (TM), sickle cell disease (SCD), severe aplastic anemia, Fanconi's anemia, dyskeratosis congenita, Blackfan-Diamond anemia, Thalassemia, congenital amegakaryocytic thrombocytopenia, severe combined immunodeficiency, T cell immunodeficiency, T cell immunodeficiency-SCID variants, Wiskott-Aldrich syndrome, a hemophagocytic disorder, a lymphoproliferative disorder, severe congenital neutropenia, chronic granulomatous disease, a phagocytic cell disorder, IPEX syndrome, juvenile rheumatoid arthritis, systemic sclerosis, an autoimmune disorder, an immune dysregulation disorder, mucopolysaccharoidoses, MPS-I, MPS-VI, osteopetrosis, a metabolic disease, globoid cell leukodystrophy (Krabbe), metachromatic leukodystrophy, cerebral X-linked adrenoleukodystrophy, a myelofibrosis disease, a myeloproliferative disease, a plasma cell disorder, a mast cell disease, common variable immunodeficiency, chronic granulomatous disease, multiple sclerosis, systemic sclerosis, rheumatoid arthritis, systemic lupus erythematosus, Crohn's disease and polymyositis-dermatomyositis. For example, the subject can be diagnosed with a disease or disorder and be designated to undergo an allogeneic transplant. For example, the subject can be diagnosed with a disease or disorder and be designated to undergo an autologous transplant. Alternatively, the subject can be diagnosed with a disease or disorder and has previously undergone an allogeneic transplant. Alternatively, the subject can be diagnosed with a disease or disorder and has previously undergone an autologous transplant.

In some aspects, the subject can be diagnosed with a cancer and be designated to undergo a transplant in order to treat the cancer. In some aspects, the subject can be diagnosed with a cancer and has previously undergone a transplant in order to treat the cancer.

In some aspects, the subject can be diagnosed with a cancer and be designated to undergo an allogeneic transplant in order to treat the cancer. In some aspects, the subject can be diagnosed with a cancer and has previously undergone an allogeneic transplant in order to treat the cancer.

In some aspects, the subject can be diagnosed with a cancer and be designated to undergo an autologous transplant in order to treat the cancer. In some aspects, the subject can be diagnosed with a cancer and has previously undergone an autologous transplant in order to treat the cancer.

In some aspects, the subject can be diagnosed with a disease or disorder and be designated to undergo a transplant in order to treat the disease or disorder. In some aspects, the subject can be diagnosed with a disease or disorder and has previously undergone a transplant in order to treat the disease or disorder.

In some aspects, the subject can be diagnosed with a disease or disorder and be designated to undergo an allogeneic transplant in order to treat the disease or disorder. In some aspects, the subject can be diagnosed with a disease or disorder and has previously undergone an allogeneic transplant in order to treat the disease or disorder.

In some aspects, the subject can be diagnosed with a disease or disorder and be designated to undergo an autologous transplant in order to treat the disease or disorder. In some aspects, the subject can be diagnosed with a disease or disorder and has previously undergone an autologous transplant in order to treat the disease or disorder.

The subject can have been previously administered a transplant. Accordingly, apraglutide, or a pharmaceutically acceptable salt thereof, can be administered to the subject after the latter has been administered a transplant.

The subject can have been previously administered an allogeneic transplant. Accordingly, apraglutide, or a pharmaceutically acceptable salt thereof, can be administered to the subject after the latter has been administered an allogeneic transplant.

The subject can have been previously administered an autologous transplant. Accordingly, apraglutide, or a pharmaceutically acceptable salt thereof, can be administered to the subject after the subject has been administered an autologous transplant.

The subject can have been previously administered a conditioning therapy in connection with a transplant. For example, the subject can have been previously administered a conditioning therapy in connection with an allogeneic transplant or in connection with an autologous transplant. A conditioning therapy can comprise the administration of radiation therapy, chemotherapy, radiomimetic therapy or any combination thereof. A radiation therapy can comprise total body irradiation.

A conditioning therapy may be administered in connection with the allogenic transplant. A conditioning therapy can comprise, such as consist of, the administration of radiation therapy, chemotherapy, radiomimetic therapy or any combination thereof. The radiation therapy can comprise total body irradiation.

Accordingly, in some aspects, an at least one therapeutically effective amount of apraglutide can be administered to the subject after the subject has been administered a conditioning therapy in connection with a transplant. In some aspects, an at least one therapeutically effective amount of apraglutide can be administered to the subject after the subject has been administered a conditioning therapy in connection with an allogeneic transplant. In some aspects, an at least one therapeutically effective amount of apraglutide can be administered to the subject after the subject has been administered a conditioning therapy in connection with an autologous transplant. In some aspects, an at least one therapeutically effective amount of apraglutide can be administered to a subject after the subject has been administered a radiation therapy, chemotherapy, radiomimetic therapy or any combination thereof.

Accordingly, apraglutide, or a pharmaceutically acceptable salt thereof, can be administered to the subject after the subject has been administered a conditioning therapy in connection with a transplant. Accordingly, apraglutide, or a pharmaceutically acceptable salt thereof, can be administered to the subject after the subject has been administered a conditioning therapy in connection with an allogeneic transplant. Accordingly, apraglutide, or a pharmaceutically acceptable salt thereof, can be administered to the subject after the subject has been administered a conditioning therapy in connection with an autologous transplant.

In some aspects, at least one therapeutically effective amount of apraglutide can be administered to a subject prior to the subject being administered a transplant. In some aspects, , at least one therapeutically effective amount of apraglutide can be administered prior the subject being administered radiation therapy, chemotherapy, or radiomimetic therapy in connection with the transplant. In some aspects, , at least one therapeutically effective amount of apraglutide is administered to the subject after the subject has been administered radiation therapy, chemotherapy, radiomimetic therapy or any combination thereof and prior to the subject being administered a transplant.

In some aspects, apraglutide can be administered to a subject prior to the subject being administered a transplant. In some aspects, apraglutide can be administered prior the subject being administered radiation therapy, chemotherapy, or radiomimetic therapy in connection with the transplant. In some aspects, apraglutide is administered to the subject after the subject has been administered radiation therapy, chemotherapy, radiomimetic therapy or any combination thereof and prior to the subject being administered a transplant.

In some aspects, apraglutide can be administered to a subject prior to the subject being administered a solid organ transplant. In some aspects, apraglutide can be administered prior the subject being administered radiation therapy, chemotherapy, or radiomimetic therapy in connection with the solid organ transplant. In some aspects, apraglutide is administered to the subject after the subject has been administered radiation therapy, chemotherapy, radiomimetic therapy or any combination thereof and prior to the subject being administered a solid organ transplant.

In some aspects, at least one therapeutically effective amount of apraglutide can be administered to a subject prior to the subject being administered an allogeneic transplant. In some aspects, at least one therapeutically effective amount of apraglutide can be administered prior the subject being administered radiation therapy, chemotherapy, or radiomimetic therapy in connection with the allogeneic transplant. In some aspects, at least one therapeutically effective amount of apraglutide is administered to the subject after the subject has been administered radiation therapy, chemotherapy, radiomimetic therapy or any combination thereof and prior to the subject being administered an allogeneic transplant.

In some aspects, apraglutide can be administered to a subject prior to the subject being administered an allogeneic transplant. In some aspects, apraglutide can be administered prior the subject being administered radiation therapy, chemotherapy, or radiomimetic therapy in connection with the allogeneic transplant. In some aspects, apraglutide is administered to the subject after the subject has been administered radiation therapy, chemotherapy, radiomimetic therapy or any combination thereof and prior to the subject being administered an allogeneic transplant.

In some aspects, at least one therapeutically effective amount of apraglutide can be administered to a subject prior to the subject being administered an autologous transplant. In some aspects, at least one therapeutically effective amount of apraglutide can be administered prior the subject being administered radiation therapy, chemotherapy, or radiomimetic therapy in connection with the autologous transplant. In some aspects, at least one therapeutically effective amount of apraglutide is administered to the subject after the subject has been administered radiation therapy, chemotherapy, radiomimetic therapy or any combination thereof and prior to the subject being administered an autologous transplant.

In the present disclosure, apraglutide, or a pharmaceutically acceptable salt thereof, can be administered to the subject prior to the subject being administered an allogeneic transplant. For example this can be prior the subject being administered radiation therapy, chemotherapy, radiomimetic therapy or any combination thereof in connection with the allogeneic transplant or after the subject has been administered radiation therapy, chemotherapy, radiomimetic therapy or any combination thereof but prior to the subject being administered an allogeneic transplant.

In the present disclosure, apraglutide, or a pharmaceutically acceptable salt thereof, can be administered to the subject prior to the subject being administered an autologous transplant. For example this can be prior the subject being administered radiation therapy, chemotherapy, radiomimetic therapy or any combination thereof in connection with the autologous transplant or after the subject has been administered radiation therapy, chemotherapy, radiomimetic therapy or any combination thereof but prior to the subject being administered an autologous transplant.

In some aspects, at least one therapeutically effective amount of apraglutide can be administered to a subject concurrently with a transplant. In some aspects, of the methods and uses of the present disclosure, an at least one therapeutically effective amount of apraglutide can be administered to a subject concurrently with an allogeneic transplant. In some aspects, at least one therapeutically effective amount of apraglutide can be administered to a subject concurrently with an autologous transplant.

In the present disclosure, apraglutide, or a pharmaceutically acceptable salt thereof, can be administered to the subject concurrently with a transplant. In the present disclosure, apraglutide, or a pharmaceutically acceptable salt thereof, can be administered to the subject concurrently with an allogeneic transplant. In the present disclosure, apraglutide, or a pharmaceutically acceptable salt thereof, can be administered to the subject concurrently with an autologous transplant.

In some aspects, at least one therapeutically effective amount of apraglutide can be administered to a subject concurrently with a conditioning therapy. In some aspects, at least one therapeutically effective amount of apraglutide least one therapeutically effective amount of apraglutide can be administered to a subject concurrently with a radiation therapy, a chemotherapy, a radiomimetic therapy, or any combination thereof.

In the present disclosure, apraglutide, or a pharmaceutically acceptable salt thereof, can be administered to the subject concurrently with a conditioning therapy, such as a conditioning therapy as defined above.

In some aspects, the subject can have been previously diagnosed with GvHD and undergone a preliminary GvHD treatment. In some aspects, the preliminary GvHD treatment can comprise the administration of an anti-GvHD treatment. In some aspects, an anti-GvHD treatment can comprise the administration a steroid therapy. Accordingly, in some aspects, the a subject can have been previously administered steroid therapy. As would be appreciated by the skilled artisan, steroid therapy can comprise the administration of at least one corticosteroid. As would be appreciated by the skilled artisan, steroid therapy can comprise the administration of prednisone, methylprednisolone, dexamethasone, beclomethasone, budesonide or any combination thereof.

Administration of apraglutide can prevent and/or attenuate a reduction in colon length in a subject following an allogeneic transplant. In some aspects, administration of apraglutide prevents and/or attenuates a reduction in colon length in a subject following a conditioning therapy and a transplant. In some aspects, administration of apraglutide prevents and/or attenuates a reduction in colon length in a subject following a conditioning therapy and an allogeneic transplant. In some aspects, administration of apraglutide prevents and/or attenuates a reduction in colon length in a subject following a conditioning therapy and an autologous transplant.

The administration of apraglutide according to the present disclosure can prevent and/or attenuate a reduction in colon length in a subject following a transplant. The administration of apraglutide may prevent and/or attenuate a reduction in colon length in a subject following a conditioning therapy and an allogeneic transplant. The administration of apraglutide according to the present disclosure can prevent and/or attenuate a reduction in colon length in a subject following an allogeneic transplant. The administration of apraglutide may prevent and/or attenuate a reduction in colon length in a subject following a conditioning therapy and an autologous transplant.

The administration of apraglutide according to the present disclosure can stabilize the intestinal microbiota, as is described in the examples herein.

Also disclosed herein is a method of treating GvHD-induced intestinal damage in a subject who has been previously diagnosed with GvHD, the method comprising administering to the subject at least one therapeutically effective amount of apraglutide, or a pharmaceutically acceptable salt thereof. The subject may have been previously diagnosed with GvHD and the GvHD is ongoing at the time of the administration of apraglutide. Alternatively, the subject may have been previously diagnosed with GvHD and the GvHD has been successfully treated prior to the administration of apraglutide. Disclosed herein is a method of treating GvHD-induced intestinal damage in a subject, the method comprising administering to the subject a therapeutically effective amount of apraglutide prior to, concurrent with, or after administration of a therapeutically effective amount of an immunosuppressive therapy. The immunosuppressive therapy may be, for example, ruxolitinib, systemic corticosteroid. Also disclosed herein is a method of treating GvHD-induced intestinal damage in a subject, the method comprising administering to the subject a therapeutically effective amount of apraglutide prior to, concurrent with, or after administration of a therapeutically effective amount of a calcineurin inhibitor.

Also disclosed herein is a method of treating GvHD-induced intestinal damage in a subject who has been previously diagnosed with GvHD, the method comprising administering to the subject apraglutide, or a pharmaceutically acceptable salt thereof. The subject may have been previously diagnosed with GvHD and the GvHD is ongoing at the time of the administration of apraglutide. Alternatively, the subject may have been previously diagnosed with GvHD and the GvHD has been successfully treated prior to the administration of apraglutide. Disclosed herein is a method of treating GvHD-induced intestinal damage in a subject, the method comprising administering to the subject apraglutide prior to, concurrent with, or after administration of a therapeutically effective amount of an immunosuppressive therapy. The immunosuppressive therapy may be, for example, ruxolitinib, systemic corticosteroid. Also disclosed herein is a method of treating GvHD-induced intestinal damage in a subject, the method comprising administering to the subject apraglutide prior to, concurrent with, or after administration of a therapeutically effective amount of a calcineurin inhibitor.

Also disclosed herein is a method of treating cancer comprising administering to a subject a combination of apraglutide, or a pharmaceutically acceptable salt thereof, and at least one chemotherapy.

In some aspects, the administration of apraglutide, or a pharmaceutically acceptable salt thereof, can treat chemotherapy-induced intestinal damage in a subject. Accordingly, also disclosed herein is a method of treating chemotherapy-induced intestinal damage in a subject in need thereof, the method comprising administering to the subject at least one therapeutically effective amount of apraglutide, or a pharmaceutically acceptable salt thereof. Also disclosed herein is a method of treating chemotherapy-induced intestinal damage in a subject in need thereof, the method comprising administering to the subject apraglutide, or a pharmaceutically acceptable salt thereof. Also disclosed herein is apraglutide, or a pharmaceutically acceptable salt thereof, for use in the treatment of chemotherapy-induced intestinal damage in a subject.

Also disclosed herein is a method of treating cancer in a subject, the method comprising administering to the subject a therapeutically effective amount of apraglutide prior to, concurrent with, or after administration of a therapeutically effective amount of a conditioning therapy. Also disclosed herein is a method of treating cancer in a subject, the method comprising administering to the subject apraglutide prior to, concurrent with, or after administration of a therapeutically effective amount of a conditioning therapy. In some aspects, the conditioning therapy is, for example, chemotherapy. In some aspects, the chemotherapy comprises a combination therapy.

Also disclosed herein is a method of treating cancer in a subject, the method comprising administering to the subject: a) a therapeutically effective amount of apraglutide; and b) a transplant. Also disclosed herein is a method of treating cancer in a subject, the method comprising administering to the subject: a) a therapeutically effective amount of a conditioning therapy; b) a therapeutically effective amount of apraglutide; and c) a transplant. Also disclosed herein is a method of treating cancer in a subject, the method comprising administering to the subject: a) a therapeutically effective amount of a radiation therapy, chemotherapy, radiomimetic therapy or any combination thereof; b) a therapeutically effective amount of apraglutide; and c) a transplant.

Also disclosed herein is a method of treating cancer in a subject, the method comprising administering to the subject: a) apraglutide; and b) a transplant. Also disclosed herein is a method of treating cancer in a subject, the method comprising administering to the subject: a) a conditioning therapy; b) apraglutide; and c) a transplant. Also disclosed herein is a method of treating cancer in a subject, the method comprising administering to the subject: a) radiation therapy, chemotherapy, radiomimetic therapy or any combination thereof; b) apraglutide; and c) a transplant.

Also disclosed herein is a method of treating cancer in a subject, the method comprising administering to the subject: a) a therapeutically effective amount of apraglutide; and b) an allogeneic transplant. Also disclosed herein is a method of treating cancer in a subject, the method comprising administering to the subject: a) a therapeutically effective amount of a conditioning therapy; b) a therapeutically effective amount of apraglutide; and c) an allogeneic transplant. Also disclosed herein is a method of treating cancer in a subject, the method comprising administering to the subject: a) a therapeutically effective amount of a radiation therapy, chemotherapy, radiomimetic therapy or any combination thereof; b) a therapeutically effective amount of apraglutide; and c) an allogeneic transplant.

Also disclosed herein is a method of treating cancer in a subject, the method comprising administering to the subject: a) apraglutide; and b) an allogeneic transplant. Also disclosed herein is a method of treating cancer in a subject, the method comprising administering to the subject: a) conditioning therapy; b) apraglutide; and c) an allogeneic transplant. Also disclosed herein is a method of treating cancer in a subject, the method comprising administering to the subject: a) radiation therapy, chemotherapy, radiomimetic therapy or any combination thereof; b) apraglutide; and c) an allogeneic transplant.

Also disclosed herein is a method of treating cancer in a subject, the method comprising administering to the subject: a) a therapeutically effective amount of apraglutide; and b) an autologous transplant. The present disclosure provides a method of treating cancer in a subject, the method comprising administering to the subject: a) a therapeutically effective amount of a conditioning therapy; b) a therapeutically effective amount of apraglutide; and c) an autologous transplant. Also disclosed herein is a method of treating cancer in a subject, the method comprising administering to the subject: a) a therapeutically effective amount of a radiation therapy, chemotherapy, radiomimetic therapy or any combination thereof; b) a therapeutically effective amount of apraglutide; and c) an autologous transplant.

Also disclosed herein is a method of treating cancer in a subject, the method comprising administering to the subject: a) apraglutide; and b) an autologous transplant. Also disclosed herein is a method of treating cancer in a subject, the method comprising administering to the subject: a) conditioning therapy; b) apraglutide; and c) an autologous transplant. Also disclosed herein is a method of treating cancer in a subject, the method comprising administering to the subject: a) radiation therapy, chemotherapy, radiomimetic therapy or any combination thereof; b) apraglutide; and c) an autologous transplant.

The presently disclosed effect of apraglutide on GvHD can be useful in the context of an anticancer therapy. GvHD developing in this context can also be referred to as anticancer therapy-related GvHD. A typical anticancer therapy that can benefit from the presently disclosed effect of apraglutide is one that includes the administration of an allogeneic or autologous transplant to a subject, such as the administration of a conditioning therapy and the subsequent administration of an allogeneic or autologous transplant. In this context, it may be advantageous to further administrate apraglutide. For example, apraglutide can be administered before the administration of an allogeneic or autologous transplant, such between the administration of a conditioning therapy and the administration of an allogeneic or autologous transplant to a subject.

Accordingly, the present disclosure provides apraglutide, or a pharmaceutically acceptable salt thereof, for use in the treatment or prevention of anticancer therapy-related acute gastrointestinal GvHD in a subject.

Also disclosed herein is a method of treating a disease or disorder in a subject, the method comprising administering to the subject: a) a therapeutically effective amount of apraglutide; and b) a transplant. Also disclosed herein is a method of treating a disease or disorder in a subject, the method comprising administering to the subject: a) a therapeutically effective amount of a conditioning therapy; b) a therapeutically effective amount of apraglutide; and c) a transplant. Also disclosed herein is s a method of treating a disease or disorder in a subject, the method comprising administering to the subject: a) a therapeutically effective amount of a radiation therapy, chemotherapy, radiomimetic therapy or any combination thereof; b) a therapeutically effective amount of apraglutide; and c) a transplant.

Also disclosed herein is a method of treating a disease or disorder in a subject, the method comprising administering to the subject: a) apraglutide; and b) a transplant. Also disclosed herein is a method of treating a disease or disorder in a subject, the method comprising administering to the subject: a) a conditioning therapy; b) apraglutide; and c) a transplant. Also disclosed herein is a method of treating a disease or disorder in a subject, the method comprising administering to the subject: a) radiation therapy, chemotherapy, radiomimetic therapy or any combination thereof; b) apraglutide; and c) a transplant.

Also disclosed herein is a method of treating a disease or disorder in a subject, the method comprising administering to the subject: a) a therapeutically effective amount of apraglutide; and b) an allogeneic transplant. Also disclosed herein is a method of treating a disease or disorder in a subject, the method comprising administering to the subject: a) a therapeutically effective amount of a conditioning therapy; b) a therapeutically effective amount of apraglutide; and c) an allogeneic transplant. Also disclosed herein is a method of treating a disease or disorder in a subject, the method comprising administering to the subject: a) a therapeutically effective amount of a radiation therapy, chemotherapy, radiomimetic therapy or any combination thereof; b) a therapeutically effective amount of apraglutide; and c) an allogeneic transplant.

Also disclosed herein is a method of treating a disease or disorder in a subject, the method comprising administering to the subject: a) apraglutide; and b) an allogeneic transplant. Also disclosed herein is a method of treating a disease or disorder in a subject, the method comprising administering to the subject: a) conditioning therapy; b) apraglutide; and c) an allogeneic transplant. Also disclosed herein is a method of treating a disease or disorder in a subject, the method comprising administering to the subject: a) radiation therapy, chemotherapy, radiomimetic therapy or any combination thereof; b) apraglutide; and c) an allogeneic transplant.

Also disclosed herein is a method of treating a disease or disorder in a subject, the method comprising administering to the subject: a) a therapeutically effective amount of apraglutide; and b) an autologous transplant. Also disclosed herein is a method of treating a disease or disorder in a subject, the method comprising administering to the subject: a) a therapeutically effective amount of a conditioning therapy; b) a therapeutically effective amount of apraglutide; and c) an autologous transplant. Also disclosed herein is a method of treating a disease or disorder in a subject, the method comprising administering to the subject: a) a therapeutically effective amount of a radiation therapy, chemotherapy, radiomimetic therapy or any combination thereof; b) a therapeutically effective amount of apraglutide; and c) an autologous transplant.

Also disclosed herein is a method of treating a disease or disorder in a subject, the method comprising administering to the subject: a) apraglutide; and b) an autologous transplant. Also disclosed herein is a method of treating a disease or disorder in a subject, the method comprising administering to the subject: a) conditioning therapy; b) apraglutide; and c) an autologous transplant. Also disclosed herein is a method of treating a disease or disorder in a subject, the method comprising administering to the subject: a) radiation therapy, chemotherapy, radiomimetic therapy or any combination thereof; b) apraglutide; and c) an autologous transplant.

In any of the methods of uses described herein, reference to apraglutide also encompasses a pharmaceutically acceptable salt of apraglutide.

In some aspects, a subject can have been previously administered ruxolitinib prior to the administration of apraglutide.

In any of the methods or uses described here, apraglutide, or a pharmaceutically acceptable salt thereof, can be administered in combination with at least one second active agent. In some aspects, the at least one second active agent can be ruxolitinib. In some aspects, the at least one second active agent can be a calcineurin inhibitor.

In some aspects of the methods and uses described herein, apraglutide, or a pharmaceutically acceptable salt thereof can be administered in temporal proximity with another therapeutic intervention described herein (*e.g.* a transplant, a conditioning therapy, chemotherapy, a second active agent, etc.).

In some aspects, of the methods and uses of the present disclosure, a transplant can comprise hematopoietic stem cells. In some aspects, the hematopoietic stem cells can be derived from bone marrow, peripheral blood, umbilical cord blood or any combination thereof. In some aspects, of the methods and uses of the present disclosure, a transplant can be an allogeneic transplant or an autologous transplant.

In some aspects, of the methods and uses of the present disclosure, an allogeneic transplant can comprise allogeneic hematopoietic stem cells. In some aspects, the allogeneic hematopoietic stem cells can be derived from bone marrow, peripheral blood, umbilical cord blood or any combination thereof.

In some aspects, of the methods and uses of the present disclosure, an autologous transplant can comprise autologous hematopoietic stem cells. In some aspects, the autologous hematopoietic stem cells can be derived from bone marrow, peripheral blood, umbilical cord blood or any combination thereof.

In some aspects, of the methods and uses of the present disclosure, a transplant can comprise T-cells. In some aspects, T-cells can be chimeric antigen receptor (CAR) T-cells.

In some aspects, of the methods and uses of the present disclosure, an allogeneic transplant can comprise allogeneic T-cells. In some aspects, allogeneic T-cells can be allogenic chimeric antigen receptor (CAR) T-cells. In some aspects, of the methods and uses of the present disclosure, an autologous transplant can comprise autologous T-cells. In some aspects, autologous T-cells can be autologous chimeric antigen receptor (CAR) T-cells.

In the present disclosure, an allogeneic transplant can comprise the transplant of allogeneic hematopoietic stem cells. The allogeneic hematopoietic stem cells can be derived from bone marrow, peripheral blood, umbilical cord blood or any combination thereof.

In the present disclosure, an allogeneic transplant can comprise the transplant of allogeneic T-cells. Allogeneic T-cells can be allogenic chimeric antigen receptor (CAR) T-cells.

In the present disclosure, an autologous transplant can comprise the transplant of autologous hematopoietic stem cells. The autologous hematopoietic stem cells can be derived from bone marrow, peripheral blood, umbilical cord blood or any combination thereof.

In the present disclosure, an autologous transplant can comprise the transplant of autologous T-cells. Autologous T-cells can be allogenic chimeric antigen receptor (CAR) T-cells.

In some aspects, of the methods and uses of the present disclosure, the pharmaceutically acceptable salt of apraglutide can be the sodium salt of apraglutide.

In the present disclosure, the pharmaceutically acceptable salt of apraglutide can be a base addition salt, such as the sodium salt of apraglutide.

In some aspects, of the methods and uses of the present disclosure, apraglutide can be administered as part of a pharmaceutical composition. Pharmaceutical compositions of apraglutide can comprise any pharmaceutically acceptable carrier and/or excipient. Non-limiting examples of pharmaceutically acceptable carriers and/or excipients include mannitol, glycine, L-histidine or any combination thereof.

Accordingly, in some aspects of the methods and uses described herein, apraglutide, or a pharmaceutically acceptable salt thereof, is administered as part of a pharmaceutical composition, wherein the pharmaceutical comprises apraglutide, or a pharmaceutically acceptable salt thereof, mannitol, glycine and L-histidine.

In the present disclosure, apraglutide can be part of a pharmaceutical composition. Pharmaceutical compositions can further comprise any pharmaceutically acceptable carrier and/or excipient. Non-limiting examples of pharmaceutically acceptable carriers and/or excipients include mannitol, glycine, L-histidine or any combination thereof.

### Definitions

Unless explicitly indicated otherwise, the term "apraglutide", refers to the compound which has the following structure:

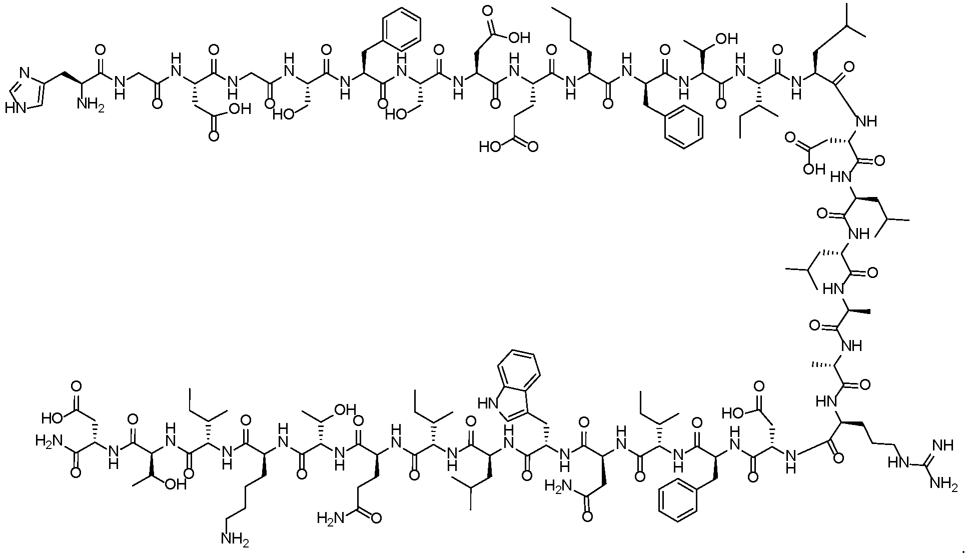

As would be appreciated by the skilled artisan, apraglutide is a GLP-2 agonist having an amino acid sequence of His-Gly-Asp-Gly-Ser-Phe-Ser-Asp-Glu-Nle-D-Phe-Thr-Ile-Leu-Asp-Leu-Leu-Ala-Ala-Arg-Asp-Phe-Ile-Asn-Trp-Leu-Ile-Gln-Thr-Lys-Ile-Thr-Asp (SEQ ID NO: 1), wherein Nle is norleucine and D-Phe is the D-amino acid phenylalanine.

Apraglutide, and its preparation, are disclosed in PCT Application Publication No. WO2011/050174, US Patent No. 8,580,918, US Patent Application Publication No. 2022-0000985 A1 and PCT Application Publication No. WO2021/252659.

Unless explicitly indicated otherwise, the terms "approximately" and "about" are synonymous. In some aspects, "approximately" and "about" refer to the recited amount, value, or duration ± 5%, ± 4.5%, ± 4%, ±3.5%, ±3%, ±2.5%, ±2%, ±1.75%, ±1.5%, ±1.25%, ±1%, ±0.9%, ±0.8%, ±0.7%, ±0.6%, ± 0.5% ±0.4%, ±0.3%, ±0.2%, ±0.1%, ±0.09%, ±0.08%, ±0.07%, ±0.06%, ±0.05%, ±0.04%, ±0.03%, ±0.02%, or ±0.01%. In some aspects, "approximately" and "about" refer to the listed amount, value, or duration ±2.5%, ±2%, ±1.75%, ±1.5%, ±1.25%, ±1%, ±0.9%, ±0.8%, ±0.7%, ±0.6%, ± 0.5%. In some aspects, "approximately" and "about" refer to the listed amount, value, or duration ±1%. In some aspects, "approximately" and "about" refer to the listed amount, value, or duration ±0.5%. In some aspects, "approximately" and "about" refer to the listed amount, value, or duration ±0.1%.

As would be appreciated by the skilled artisan, the terms "Graft versus Host Disease" or "GvHD" refer to the condition that occurs in a subject following an allogeneic or autologous transplant (e.g. a HSCT) in which immune cells presented in the allogeneic or autologous transplant material (referred to as the "graft") attack the transplant recipient's own tissues. As would be appreciated by the skilled artisan, GvHD may be classified using MAGIC grading scale as described in Biol Blood Marrow Transplant. 2016;22(1):4-10*.*

As would be appreciated by the skilled artisan, the terms "acute Graft versus Host Disease", "acute GvHD", "classic acute Graft versus Host Disease" and classic acute GvHD" refer to Graft versus Host Disease that develops in a transplant recipient within about 100 days following transplantation and manifests with clinical features that are typically associated with acute Graft versus Host Disease, including, but not limited to inflammation and tissue damage in the skin, oral and genital mucosa, eyes, gut, liver, lungs, joints, and muscle.

As would be appreciated by the skilled artisan, the terms "late acute Graft versus Host Disease" or "late acute GvHD" refer to the conditions of persistent acute Graft versus Host Disease, recurrent acute Graft versus Host Disease, and/or new-onset acute Graft versus Host Disease, which are forms of Graft versus Host Disease that manifest with the clinical features of acute Graft versus Host Disease, but more than 100 days after the transplantation.

As would be appreciated by the skilled artisan, the terms "acute gastrointestinal Graft versus Host Disease" and "acute gastrointestinal GvHD" refer to any form of acute GvHD described above that manifests in a subject with gastrointestinal symptoms and/or damage, including, but not limited to, diarrhea, abdominal pain, mucositis, mucosal ulceration, nausea, shortening of the colon, shortening of the small intestine.

As would be appreciated by the skilled artisan, the term "steroid-refractory", "steroid-refractory GvHD", and "SR-GvHD" refers to GvHD that has been previously treated using steroid therapy, but has become non-responsive to the steroid therapy. Thus, in a non-limiting example, steroid-refractory acute gastrointestinal GvHD refers to acute gastrointestinal GvHD that has been previous treated using a steroid therpay, but has become non-responsive to steroid therapy. In some aspects, subjects with steroid-refractory GvHD are administered a pharmaceutical composition comprising ruxolitinib. In some aspects, subjects with steroid-refractory GvHD are administered a pharmaceutical composition comprising apraglutide. In some aspects, to a subject with steroid-refractory GvHD is administered a therapeutically effective amount of apraglutide prior to, concurrent with, or after administration of a therapeutically effective amount of immunosuppressive therapy. In some aspects, to a subject with steroid-refractory GvHD is administered a therapeutically effective amount of apraglutide prior to, concurrent with, or after administration of a therapeutically effective amount of ruxolitinib and/or systemic steroids.

As would be appreciated by the skilled artisan, the term "steroid-naive" refers to GvHD that has not been previously treated using a steroid therapy. Thus, in a non-limiting example, steroid-naive acute gastrointestinal GvHD refers to acute gastrointestinal GvHD that has not been previously treated using a steroid therapy.

As would be appreciated by the skilled artisan, the terms "chronic Graft versus Host Disease" or "chronic GvHD" refer to Graft versus Host Disease that develop more than 100 days after the transplantation and manifests with clinical features that are typically associated with chronic Graft versus Host Disease, including, but not limited to, damage to connective tissue of exocrine glands, tissue fibrosis and limitation of joint motility, fibrosis of the lungs and liver, immune dysregulation and autoimmunity.

As would be appreciated by the skilled artisan, the term "chronic gastrointestinal Graft versus Host Disease" and "chronic gastrointestinal GvHD" refer to any form of chronic GvHD described above that manifests in a subject with gastrointestinal symptoms and/or damage, including, but not limited to, diarrhea, abdominal pain, mucositis, mucosal ulceration, nausea, shortening of the colon, shortening of the small intestine.

As would be appreciated by the skilled artisan, the term "Overlap Syndrome" refers to Graft versus Host Disease that can present at any time post-transplant and that manifests with clinical features of both acute Graft versus Host Disease and chronic Graft versus Host Disease.

As would be appreciated by the skilled artisan, the terms "upper gut Graft versus Host Disease", " upper gut GvHD", " upper gut GvHD phenotype" and "upper gut phenotype of GvHD " refer to Graft versus Host Disease that presents with persistent loss of appetite, satiety, nausea, vomiting, and weight loss, with variable amounts of diarrhea. The presentation can be indolent, and therapy with prednisone at doses of 1 mg/kg/day plus topical oral corticosteroid is effective. In some aspects, the upper gut GvHD phenotype does not progress to the mid-lower gut GvHD phenotype. In some aspects, the upper gut GvHD phenotype is limited to GvHD grade I.

As would be appreciated by the skilled artisan, the terms " mid-lower gut Graft versus Host Disease", "mid-lower gut GvHD", "mid-lower gut GvHD phenotype" and "mid-lower gut phenotype of GvHD " refer to Graft versus Host Disease that presents with secretory, protein-rich and bile salt diarrhea and abdominal pain resulting from gut distention. In some aspects, mid-lower gut GvHD is severe, wherein the subject presents with the entire small intestine and colon as edematous and inflamed, with larger diarrheal volumes and evidence of mucosal ulceration and bleeding. In some aspects, subjects with severe mid-lower gut GvHD require prolonged hospitalization for supportive care including total parenteral nutrition and pain control. In some aspects, subjects with severe mid-lower gut GvHD require the standard initial therapy comprising prednisone with or without other immune suppressive therapies.

As would be appreciated by the skilled artisan, the term "GI-aGVHD response" refers to a decrease of one stage in the signs and symptoms of GI-aGVHD without any intercurrent events of discontinuation of the assigned apraglutide treatment, institution of new systemic therapy, or death. In some aspects, a complete GI-aGVHD response refers to the resolution of all GI-aGVHD signs and symptoms GVHD without any intercurrent events of discontinuation of the assigned apraglutide treatment, institution of new systemic therapy, or death. In some aspects, a partial response refers to an improvement of 1 stage in one or more organs involved with aGVHD signs or symptoms without progression in other organs or sites without administration of additional systemic therapies for an earlier progression, mixed response or non-response of aGVHD. In some aspects, a GI-aGVHD flare refers to any increase in signs or symptoms of GI-aGVHD that is sustained for >24 h after an initial response and requires re-escalation of immunosuppressive therapy (e.g., corticosteroid, calcineurin inhibitors, and/or ruxolitinib dosing).

The term "subject" includes any living organism that has GvHD, or is at a risk of developing GvHD. In some aspects, the term "subject" refers to a mammal that has GvHD, or is at a risk of developing GvHD. In some aspects, the term subject refers to a human being that has GvHD, or is at a risk of developing GvHD. A human that is at risk of developing GvHD can be a human that is to receive, is currently receiving, or has previously received an transplant (allogeneic or autologous). A human that is at risk of developing GvHD can be a human that is to receive, is currently receiving, or has previously received a conditioning therapy in connection with an HSCT.

The term "patient" is meant to be synonymous and may be used interchangeably with "subject," unless explicitly indicated otherwise.

The term "allogeneic" refers to biological material isolated from a donor that is to be transplanted into a recipient, wherein the donor and the recipient are two different subjects.

The term "autologous" refers to biological material isolated from a donor that is to be transplanted into a recipient, wherein the donor and the recipient is the same subject.

The term "conditioning therapy" refers to the use of radiation therapy, such as total body irradiation, chemotherapy, or radiomimetic therapy that typically administered to a subject prior to an HSCT. As would be appreciated by the skilled artisan, the aims of conditioning therapy include: a) eradication of the underlying disease (e.g. cancer, a blood cancer) that is to be treated using by the HSCT; b) creation of space in the bone marrow for donor stem cells to engraft; and c) immunosuppression to decrease the risk of rejection of the donor cells by the host cells. As used herein, conditioning therapy can refer to any conditioning therapy known in the art, including, but not limited to, total body irradiation. The term "radiomimetic therapy" refers to any drug, compound or treatment known in the art that imitates the effects of radiation. As would be appreciated by the skilled artisan, chemotherapy includes the administration of a therapeutically effective amount of at least one chemotherapeutic agent. In some aspects, the at least one chemotherapeutic agent may include, but it is not limited to, Actinomycin, All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Melphalan, Mercaptopurine, Methotrexate, Mitoxantrone, Oxaliplatin, Paclitaxel, Pemetrexed, Teniposide, Tioguanine, Topotecan, Valrubicin, Vemurafenib, Vinblastine, Vincristine, Vindesine. In some aspects, the at least one chemotherapeutic agent comprises Cytarabine. In some aspects, the at least one chemotherapeutic agent comprises Melphalan.

As used herein, the term "treating" or "treat" describes the management and care of a subject for the purpose of combating a disease, condition, or disorder and includes the administration of a compound of the present disclosure, or a pharmaceutically acceptable salt, polymorph or solvate thereof, to alleviate the symptoms or complications of a disease, condition or disorder, or to eliminate the disease, condition or disorder. The term "treat" can also include treatment of a cell in vitro or an animal model.

It is to be appreciated that references to "treating" or "treatment" include the alleviation of established symptoms of a condition. "Treating" or "treatment" of a state, disorder or condition therefore includes: (1) delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

As used herein, the term "preventing," "prevent," or "protecting against" describes reducing or eliminating the onset of the symptoms or complications of such disease, condition or disorder.

The term "therapeutically effective amount", as used herein, refers to an amount of a pharmaceutical agent, e.g., apraglutide, to treat, or prevent an identified disease or condition, e.g., GvHD, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician.

For any compound, the therapeutically effective amount can be estimated in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between toxic and therapeutic effects is the therapeutic index, and it can be expressed as the ratio, LD₅₀/ED₅₀. The dosage may vary within this range depending upon the dosage form employed and sensitivity of the subject.

The terms "administer", "administering", "administration", and the like, as used herein, refer to methods that may be used to enable delivery of compositions to the desired site of biological action. These methods include, but are not limited to, intraarticular (in the joints), intravenous, intramuscular, intratumoral, intradermal, intraperitoneal, subcutaneous, orally, topically, intrathecally, inhalationally, transdermally, rectally, and the like. Administration techniques that can be employed with the agents and methods described herein are found in *e.g.*, Goodman and Gilman, The Pharmacological Basis of Therapeutics, current ed.; Pergamon; and Remington's, Pharmaceutical Sciences (current edition), Mack Publishing Co., Easton, Pa.

In addition, the apraglutide can be co-administered with other therapeutic agents. As used herein, the terms "co-administration", "administered in combination with", and their grammatical equivalents, are meant to encompass administration of two or more therapeutic agents to a single subject, and are intended to include treatment regimens in which the agents are administered by the same or different route of administration or at the same or different times. In some aspects, apraglutide will be co-administered with other agents. These terms encompass administration of two or more agents to the subject so that both agents and/or their metabolites are present in the subject at the same time. They include simultaneous administration in separate compositions, administration at different times in separate compositions, and/or administration in a composition in which both agents are present (i.e. co-formulation). Thus, the compounds described herein and the other agent(s) may be administered in a single composition.

The particular mode of administration and the dosage regimen will be selected by the attending clinician, taking into account the particulars of the case (*e.g.*, the subject, the disease, the disease state involved, the particular treatment). Treatment can involve daily or multi-daily or less than daily (such as weekly or monthly etc.) doses over a period of a few days to months, or even years. However, a person of ordinary skill in the art would immediately recognize appropriate and/or equivalent doses looking at dosages of apraglutide used in the treatment of other diseases and conditions, including, but not limited to, short bowel syndrome.

Doses generally range from about 1 mg to about 10 mg per week for a period of about 1 week to about 100 weeks. In some aspects, the weekly dose is between about 1 mg and 10 mg. In some aspects, subjects are dosed from between about 1 weeks to about 100 weeks, about 1 weeks to about 80 weeks, about 1 weeks to about 60 weeks, about 1 weeks to about 48 weeks, about 1 weeks to about 26 weeks, about 1 weeks to about 13 weeks, about 1 weeks to about 8 weeks, about 2 weeks to about 24 weeks, about 2 weeks to about 20 weeks, or about 2 weeks to about 16 weeks. In some aspects, subjects may be administered a weekly dose range from about 1 mg to about 30 mg. In some aspects, subjects may be administered a weekly dose range from about 1 mg to about 28.4 mg. In some aspects, subjects may be administered a once a week of about 10 mg. In some aspects, subjects may be administered a once a week of about 5 mg. In some aspects, subjects may be administered a once a week of about 2.5 mg. In some aspects, subjects may be administered a once a week of about 1 mg. For example, subjects may be administered a dose about once a week. Subjects may be administered a dose about once every two weeks or about twice a month. About once every two weeks or about twice a month. Patients with a body weight below 50 kg can be administered apraglutide in an amount of 2.5 mg to prevent high exposures. In some aspects, patients with a body weight of 50 kg or great can be administered apraglutide in an amount of 5 mg or more.

In some aspects, subjects with a body weight of 50 kg to 60 kg can be administered apraglutide in an amount from about 2.5 mg to about 5 mg. In some aspects, subjects with a body weight of 50 kg to 60 kg can be administered apraglutide in an amount of about 2.5 mg. In some aspects, subjects with a body weight of 50 kg to 60 kg can be administered apraglutide in an amount of about 5 mg.

In some aspects, subjects with a body weight of 50 kg to 60 kg can be administered apraglutide in an amount from about 2.5 mg to about 5 mg per week. In some aspects, subjects with a body weight of 50 kg to 60 kg can be administered apraglutide in an amount of about 2.5 mg per week. In some aspects, subjects with a body weight of 50 kg to 60 kg can be administered apraglutide in an amount of about 5 mg per week.

In some aspects, subjects with a body weight of 60 kg to 80 kg can be administered apraglutide in an amount from about 3.75 mg to about 7.5 mg. In some aspects, subjects with a body weight of 60 kg to 80 kg can be administered apraglutide in an amount from about 4 mg to about 7.5 mg. In some aspects, subjects with a body weight of 60 kg to 80 kg can be administered apraglutide in an amount of about 3.75 mg. In In some aspects, subjects with a body weight of 60 kg to 80 kg can be administered apraglutide in an amount of about 4 mg. In some aspects, subjects with a body weight of 60 kg to 80 kg can be administered apraglutide in an amount of about 7.5 mg.

In some aspects, subjects with a body weight of 60 kg to 80 kg can be administered apraglutide in an amount from about 3.75 mg to about 7.5 mg per week. In some aspects, subjects with a body weight of 60 kg to 80 kg can be administered apraglutide in an amount from about 4 mg to about 7.5 mg per week. In some aspects, subjects with a body weight of 60 kg to 80 kg can be administered apraglutide in an amount of about 3.75 mg. In In some aspects, subjects with a body weight of 60 kg to 80 kg can be administered apraglutide in an amount of about 4 mg per week. In some aspects, subjects with a body weight of 60 kg to 80 kg can be administered apraglutide in an amount of about 7.5 mg per week.

In some aspects, subjects with a body weight of 80 kg or great can be administered apraglutide in an amount from about 5 mg to about 10 mg. In some aspects, subjects with a body weight of 80 kg or great can be administered apraglutide in an amount of about 5 mg. In some aspects, subjects with a body weight of 80 kg or great can be administered apraglutide in an amount of about 10 mg.

In some aspects, subjects with a body weight of 80 kg or great can be administered apraglutide in an amount from about 5 mg to about 10 mg per week. In some aspects, subjects with a body weight of 80 kg or great can be administered apraglutide in an amount of about 5 mg per week. In some aspects, subjects with a body weight of 80 kg or great can be administered apraglutide in an amount of about 10 mg per week.

In some aspects, subjects may be administered a single dose range from about 1 mg to about 60 mg. In some aspects, subjects may be administered a single dose range from about 1 mg to about 56.9 mg.

In some aspects, apraglutide can be administered to a subject in an amount of at least about 0.5, or at least about 1 mg. or at least about 1.5 mg, or at least about 2 mg, or at least about 2.5 mg, or at least about 3 mg, or at least about 3.5 mg, or at least about 4 mg, or at least about 4.5 mg, or at least about 5 mg, or at least about 5.5 mg, or at least about 6 mg, or at least about 6.5 mg, or at least about 7 mg, or at least about 7.5 mg, or at least about 8 mg, or at least about 8.5 mg, or at least about 9 mg, or at least about 9.5 mg, or at least about 10 mg, or at least about 10.5 mg, or at least about 11 mg, or at least about 11.5 mg, or at least about 12 mg, or at least about 12.5 mg, or at least about 13 mg, or at least about 13.5 mg, or at least about 14 mg, or at least about 14.5 mg, or at least about 15 mg, or at least about 15.5 mg, or at least about 16 mg, or least about 16.5 mg, or at least about 17 mg, or at least about 17.5 mg, or at least about 18 mg, or at least about 18.5 mg, or at least about 19 mg, or at least about 19.5 mg, or at least about 20 mg.

In some aspects, apraglutide can be administered to a subject in an amount of about 0.5, or about 1 mg. or about 1.5 mg, or about 2 mg, or about 2.5 mg, or about 3 mg, or about 3.5 mg, or about 4 mg, or about 4.5 mg, or about 5 mg, or about 5.5 mg, or about 6 mg, or about 6.5 mg, or about 7 mg, or about 7.5 mg, or about 8 mg, or about 8.5 mg, or about 9 mg, or about 9.5 mg, or about 10 mg, or about 10.5 mg, or about 11 mg, or about 11.5 mg, or about 12 mg, or about 12.5 mg, or about 13 mg, or about 13.5 mg, or about 14 mg, or about 14.5 mg, or about 15 mg, or about 15.5 mg, or about 16 mg, or least about 16.5 mg, or about 17 mg, or about 17.5 mg, or about 18 mg, or about 18.5 mg, or about 19 mg, or about 19.5 mg, or about 20 mg.

In some aspects, apraglutide can be administered to a subject once daily, twice daily, once every two days, once every three days, once every four days, once every five days, once every six days, once every 7 days (once a week), once every 8 days, once every 9 days, once every 10 days, once every 11 days, once every 12 days, once every 13 days, once every 14 days (once every two weeks), once every 15 days, once every 16 days, once every 17 days, once every 18 days, once every 19 days, once every 20 days, once every 21 days (once every three weeks), once every 22 days, once every 23 days, once every 24 days, once every 25 days, once every 26 days, once every 27 days, once every 28 days (once every four weeks), or once a month.

A pharmaceutical composition comprising apraglutide and/or a pharmaceutically acceptable salt thereof, may further comprise at least one pharmaceutically acceptable carrier. In some aspects, the at least one pharmaceutically acceptable carrier is chosen from pharmaceutically acceptable vehicles and pharmaceutically acceptable adjuvants. In some aspects, the at least one pharmaceutically acceptable carrier is chosen from pharmaceutically acceptable fillers, disintegrants, surfactants, binders, lubricants. The term "at least one pharmaceutically acceptable carrier," as used herein, includes any and all solvents, diluents, other liquid vehicles, dispersion aids, suspension aids, surface active agents, isotonic agents, thickening agents, emulsifying agents, preservatives, solid binders, and lubricants, as suited to the particular dosage form desired. Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York discloses various carriers used in formulating pharmaceutical compositions and known techniques for the preparation thereof. Except insofar as any conventional carrier is incompatible with apraglutide, such as by producing any undesirable biological effect or otherwise interacting in a deleterious manner with any other component(s) of the pharmaceutical composition, its use is contemplated to be within the scope of this disclosure. Non-limiting examples of suitable pharmaceutically acceptable carriers include, but are not limited to, ion exchangers, alumina, aluminum stearate, lecithin, serum proteins (such as human serum albumin), buffer substances (such as phosphates, glycine, sorbic acid, and potassium sorbate), partial glyceride mixtures of saturated vegetable fatty acids, water, salts, and electrolytes (such as protamine sulfate, disodium hydrogen phosphate, potassium hydrogen phosphate, sodium chloride, and zinc salts), colloidal silica, magnesium trisilicate, polyvinyl pyrrolidone, polyacrylates, waxes, polyethylene-polyoxypropylene-block polymers, wool fat, sugars (such as lactose, glucose and sucrose), starches (such as corn starch and potato starch), cellulose and its derivatives (such as sodium carboxymethyl cellulose, ethyl cellulose and cellulose acetate), powdered tragacanth, malt, gelatin, talc, excipients (such as cocoa butter and suppository waxes), oils (such as peanut oil, cottonseed oil, safflower oil, sesame oil, olive oil, corn oil and soybean oil), glycols (such as propylene glycol and polyethylene glycol), esters (such as ethyl oleate and ethyl laurate), agar, buffering agents (such as magnesium hydroxide and aluminum hydroxide), alginic acid, pyrogen-free water, isotonic saline, Ringer's solution, ethyl alcohol, phosphate buffer solutions, non-toxic compatible lubricants (such as sodium lauryl sulfate and magnesium stearate), coloring agents, releasing agents, coating agents, sweetening agents, flavoring agents, perfuming agents, preservatives, and antioxidants. In some aspects, the at least one pharmaceutically acceptable carrier is chosen from glycine, L-Histidine, mannitol, and sodium hydroxide.

The compounds or the corresponding pharmaceutical compositions taught herein can be administered to a patient in a variety of forms depending on the selected route of administration, as will be understood by those skilled in the art. The compounds of the present teachings may be administered, for example, by oral, parenteral, buccal, sublingual, nasal, rectal, patch, pump or transdermal administration and the pharmaceutical compositions formulated accordingly. Parenteral administration includes intravenous, intraperitoneal, subcutaneous, intramuscular, transepithelial, nasal, intrapulmonary, intrathecal, rectal and topical modes of administration. Parenteral administration can be by continuous infusion over a selected period of time.

The pharmaceutical composition of the application is formulated to be compatible with its intended route of administration. The composition may be formulated in accordance with routine procedures as a pharmaceutical composition adapted for intravenous, subcutaneous, intramuscular, oral, intranasal, or topical administration to human beings. In preferred aspects, the pharmaceutical composition is formulated for intravenous administration.

The oral therapeutic administration may be incorporated with an excipient and used in the form of ingestible tablets, buccal tablets, troches, capsules, elixirs, suspensions, syrups, wafers, and the like.

The parenteral administration may be generally prepared in water suitably mixed with a surfactant such as hydroxypropylcellulose. Dispersions can also be prepared in glycerol, liquid polyethylene glycols, DMSO and mixtures thereof with or without alcohol, and in oils. Under ordinary conditions of storage and use, these preparations contain a preservative to prevent the growth of microorganisms.

The injectable administration may be prepared as a sterile aqueous solution or dispersion thereof, and sterile powders of, apraglutide for the extemporaneous preparation of sterile injectable solutions or dispersions are appropriate.

Apraglutide can be administered via subcutaneous injection. For example, apraglutide can be administered via single-dose bolus subcutaneous injection.

Apraglutide can be administered via a two-chamber syringe or dual cartridge injector. One example of such a syringe is described in PCT/EP2012/000787.

GLP-2 agonists, e.g., apraglutide, may be administered parenterally, e.g. by injection. Formulations suitable for parenteral administration include aqueous and non-aqueous, isotonic sterile injection solutions, which can contain antioxidants, buffers, bacteriostatics, and solutes that render the formulation isotonic with the blood of the intended recipient, and aqueous and non-aqueous sterile suspensions that can include suspending agents, solubilizers, thickening agents, stabilizers, and preservatives. Liquid carriers, for injectable solutions, include by way of example and without limitation water, saline, aqueous dextrose and glycols.

Apraglutide can be administered as part of a pharmaceutical composition. Pharmaceutical compositions of apraglutide can comprise any pharmaceutically acceptable carrier and/or excipient. Pharmaceutical compositions of apraglutide can comprise glycine, L-histidine, mannitol, and any combination thereof.

The terms "cancer" and "cancerous" refer to or describe the physiological condition in mammals that is typically characterized by unregulated cell growth. Included in this definition are benign and malignant cancers. Examples of cancer include but are not limited to, carcinoma, lymphoma, blastoma, sarcoma, leukemia and germ cell tumors. More particular examples of such cancers include adrenocortical carcinoma, bladder urothelial carcinoma, breast invasive carcinoma, cervical squamous cell carcinoma, endocervical adenocarcinoma, cholangiocarcinoma, colon adenocarcinoma, lymphoid neoplasm diffuse large B-cell lymphoma, esophageal carcinoma, glioblastoma multiforme, head and neck squamous cell carcinoma, kidney chromophobe, kidney renal clear cell carcinoma, kidney renal papillary cell carcinoma, acute myeloid leukemia, brain lower grade glioma, liver hepatocellular carcinoma, lung adenocarcinoma, lung squamous cell carcinoma, mesothelioma, ovarian serous cystadenocarcinoma, pancreatic adenocarcinoma, pheochromocytoma, paraganglioma, prostate adenocarcinoma, rectum adenocarcinoma, sarcoma, skin cutaneous melanoma, stomach adenocarcinoma, testicular germ cell tumors, thyroid carcinoma, thymoma, uterine carcinosarcoma, uveal melanoma. Other examples include breast cancer, lung cancer, lymphoma, melanoma, liver cancer, colorectal cancer, ovarian cancer, bladder cancer, renal cancer or gastric cancer. Further examples of cancer include neuroendocrine cancer, non-small cell lung cancer (NSCLC), small cell lung cancer, thyroid cancer, endometrial cancer, biliary cancer, esophageal cancer, anal cancer, salivary, cancer, vulvar cancer, cervical cancer, Acute lymphoblastic leukemia (ALL), Acute myeloid leukemia (AML), Adrenal gland tumors, Anal cancer, Bile duct cancer, Bladder cancer, Bone cancer, Bowel cancer, Brain tumors, Breast cancer, Cancer of unknown primary (CUP), Cancer spread to bone, Cancer spread to brain, Cancer spread to liver, Cancer spread to lung, Carcinoid, Cervical cancer, Children's cancers, Chronic lymphocytic leukemia (CLL), Chrome myeloid leukemia (CML), Colorectal cancer, Ear cancer, Endometrial cancer, Eye cancer, Follicular dendritic cell sarcoma, Gallbladder cancer, Gastric cancer, Gastro esophageal junction cancers, Germ cell tumors, Gestational trophoblastic disease (GIT)), Hairy cell leukemia, Head and neck cancer, Hodgkin lymphoma, Kaposi's sarcoma, Kidney cancer, Laryngeal cancer, Leukemia, Gastric linitis plastica, Liver cancer, Lung cancer, Lymphoma, Malignant schwannoma, Mediastinal germ cell tumors, Melanoma skin cancer, Men's cancer, Merkel cell skin cancer, Mesothelioma, Molar pregnancy, Mouth and oropharyngeal cancer, Myeloma, Nasal and paranasal sinus cancer, Nasopharyngeal cancer, Neuroblastoma, Neuroendocrine tumors, Non-Hodgkin lymphoma (NHL), Esophageal cancer, Ovarian cancer, Pancreatic cancer, Penile cancer, Persistent trophoblastic disease and choriocarcinoma, Pheochromocytoma, Prostate cancer, Pseudomyxoma peritonei, Rectal cancer. Retinoblastoma, Salivary gland cancer, Secondary' cancer, Signet cell cancer, Skin cancer, Small bowel cancer, Soft tissue sarcoma, Stomach cancer, T cell childhood non Hodgkin lymphoma (NHL), Testicular cancer, Thymus gland cancer, Thyroid cancer, Tongue cancer, Tonsil cancer, Tumors of the adrenal gland, Uterine cancer. Vaginal cancer, Vulval cancer, Wilms' tumor, Womb cancer and Gynaecological cancer. Examples of cancer also include, but are not limited to, Hematologic malignancies, Lymphoma, Cutaneous T-cell lymphoma, Peripheral T-cell lymphoma, Hodgkin's lymphoma, Non-Hodgkin's lymphoma, Multiple myeloma, Chrome lymphocytic leukemia, chronic myeloid leukemia, acute myeloid leukemia, Myelodysplastic syndromes, Myelofibrosis, Biliary tract cancer, Hepatocellular cancer, Colorectal cancer, Breast cancer, Lung cancer, Non-small cell lung cancer, Ovarian cancer, Thyroid Carcinoma, Renal Cell Carcinoma, Pancreatic cancer, Bladder cancer, skin cancer, malignant melanoma, merkel cell carcinoma, Uveal Melanoma or Glioblastoma multiforme.

In some aspects, the cancer is a carcinoma, a lymphoma, a blastoma, a sarcoma, a leukemia, a brain cancer, a breast cancer, a blood cancer, a bone cancer, a lung cancer, a skin cancer, a liver cancer, an ovarian cancer, a bladder cancer, a renal cancer, a kidney cancer, a gastric cancer, a thyroid cancer, a pancreatic cancer, an esophageal cancer, a prostate cancer, a cervical cancer, a uterine cancer, a stomach cancer, a soft tissue cancer, a laryngeal cancer, a small intestine cancer, a testicular cancer, an anal cancer, a vulvar cancer, a joint cancer, an oral cancer, a pharynx cancer or a colorectal cancer.

In some aspects, the cancer is a hematological cancer.

In some aspects, the cancer is Acute myeloid leukemia, myelodysplastic syndrome, follicular lymphoma, diffuse large B cell lymphoma, acute lymphoblastic leukemia, multiple myeloma, Hodgkin lymphoma, chronic myeloid leukemia, T cell non-Hodgkin lymphoma, lymphoblastic B cell non-Hodgkin lymphoma (non-Burkitt), Burkitt's lymphoma, anaplastic large cell lymphoma, germ cell tumor, Ewing's sarcoma, soft tissue sarcoma, neuroblastoma, Wilms' tumor, osteosarcoma, medulloblastoma, acute promyelocytic leukemia, mantle cell lymphoma, T cell lymphoma, lymphoplasmacytic lymphoma, cutaneous T cell lymphoma, plasmablastic lymphoma, chronic lymphocytic leukemia, breast cancer and renal cancer.

In some aspects, a disease or disorder is a hemoglobinopathy, a congenital hemoglobinopathy, β-Thalessemia major (TM), sickle cell disease (SCD), severe aplastic anemia, Fanconi's anemia, dyskeratosis congenita, Blackfan-Diamond anemia, Thalassemia, congenital amegakaryocytic thrombocytopenia, severe combined immunodeficiency, T cell immunodeficiency, T cell immunodeficiency-SCID variants, Wiskott-Aldrich syndrome, a hemophagocytic disorder, a lymphoproliferative disorder, severe congenital neutropenia, chronic granulomatous disease, a phagocytic cell disorder, IPEX syndrome, juvenile rheumatoid arthritis, systemic sclerosis, an autoimmune disorder, an immune dysregulation disorder, mucopolysaccharoidoses, MPS-I, MPS-VI, osteopetrosis, a metabolic disease, globoid cell leukodystrophy (Krabbe), metachromatic leukodystrophy, cerebral X-linked adrenoleukodystrophy, a myelofibrosis disease, a myeloproliferative disease, a plasma cell disorder, a mast cell disease, common variable immunodeficiency, chronic granulomatous disease, multiple sclerosis, systemic sclerosis, rheumatoid arthritis, systemic lupus erythematosus, Crohn's disease or polymyositis-dermatomyositis.

As used herein, the term "temporal proximity" refers to that administration of one therapeutic composition (e.g., apraglutide, or a pharmaceutically acceptable salt thereof) occurs within a time period before or after the administration of another therapeutic composition (e.g., a transplant, a conditioning therapy, a second active agent), such that the therapeutic effect of the one therapeutic agent overlaps with the therapeutic effect of the other therapeutic agent. In some embodiments, the therapeutic effect of the one therapeutic agent completely overlaps with the therapeutic effect of the other therapeutic agent. In some embodiments, "temporal proximity" means that administration of one therapeutic agent occurs within a time period before or after the administration of another therapeutic agent, such that there is a synergistic effect between the one therapeutic agent and the other therapeutic agent. "Temporal proximity" may vary according to various factors, including but not limited to, the age, gender, weight, genetic background, medical condition, disease history, and treatment history of the subject to which the therapeutic agents are to be administered; the disease or condition to be treated or ameliorated; the therapeutic outcome to be achieved; the dosage, dosing frequency, and dosing duration of the therapeutic agents; the pharmacokinetics and pharmacodynamics of the therapeutic agents; and the route(s) through which the therapeutic agents are administered. In some embodiments, "temporal proximity" means within 15 minutes, within 30 minutes, within an hour, within two hours, within four hours, within six hours, within eight hours, within 12 hours, within 18 hours, within 24 hours, within 36 hours, within 2 days, within 3 days, within 4 days, within 5 days, within 6 days, within a week, within 2 weeks, within 3 weeks, within 4 weeks, with 6 weeks, or within 8 weeks. In some embodiments, multiple administration of one therapeutic agent can occur in temporal proximity to a single administration of another therapeutic agent. In some embodiments, temporal proximity may change during a treatment cycle or within a dosing regimen.

### Examples

The following non-limiting examples describe experiments directed at testing the use of apraglutide for the prevention and/or treatment of acute gastrointestinal GvHD in a mouse model in which intestinal damage was induced by total body irradiation of the mice meant to simulate the conditioning regimens that are typically used prior to AHCT in human subjects.

### Example 1

In a first experiment BALB/cJ male mice were separated into two groups, Group A and Group B. Each group consisted of 10 mice.

Mice in Group A received subcutaneous injections of apraglutide in an amount of 3 mg/kg/mouse (administered in 300 µl of vehicle), every second day from Day -9 to Day +7 (Days: -9, -7, -5, -3 -1, +1, +3, +5, +7).

Mice in Group B received subcutaneous injections of 300 µl of vehicle control every second day starting from Day -9 to Day +7 (Days: -9, -7, -5, -3 -1, +1, +3, +5, +7).

On Day -1 of the experiment, all mice in Groups A and B were irradiated with 8.5 Gy using an linac x-ray source.

On Day 0 of the experiment, bone marrow and T cells from C57BL/6J mice were collected, prepared and transplanted into the mice of Group A and Group B. Mice in Group A and Group B were intravenously injected with 10 million bone marrows cells extracted from the femurs of the C57BL/6J mice and 2.5 million spleen cells extracted from the C57BL/6J mice. Mouse #3 (Group A), #13 (Group B) and #20 (Group B) were administered the cells by subcutaneous injection rather than intravenously. Mouse #5 (Group B) and #10 (Group B) received half the cells by intravenous injection and half by subcutaneous injection.

Morbidity, mortality, body weight, clinical signs (diarrhea, appearance and behavior), length of intestinal segments, and histopathology of the intestine, skin, spleen and liver were monitored in the mice of Group A and Group B. All mice were sacrificed at Day +14 at the latest.

The induction of acute GvHD was observed in the mice of Group A and Group B following the transplantations, as evidenced by weight loss and histology analysis, specifically lymphocyte infiltration.

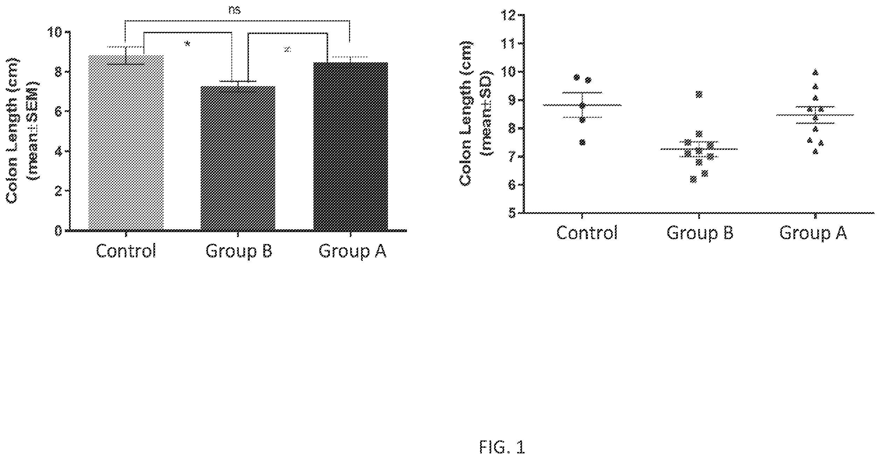

Following sacrifice of the mice, the colon lengths of the mice in Group A and Group B, as well as a control group of BALB/c mice (untreated), were measured. The results of this analysis are shown in FIG. 1. As shown in FIG. 1, there was a clear difference in colon lengths in Group A as compared to Group B, as the mice that were treated with apraglutide showed increased colon lengths that were nearly the same as the control mice. Thus, treatment with apraglutide protected the mice in Group A from a reduction in colon length, which is typically attributed to the inflammation caused by acute, gastrointestinal GvHD.

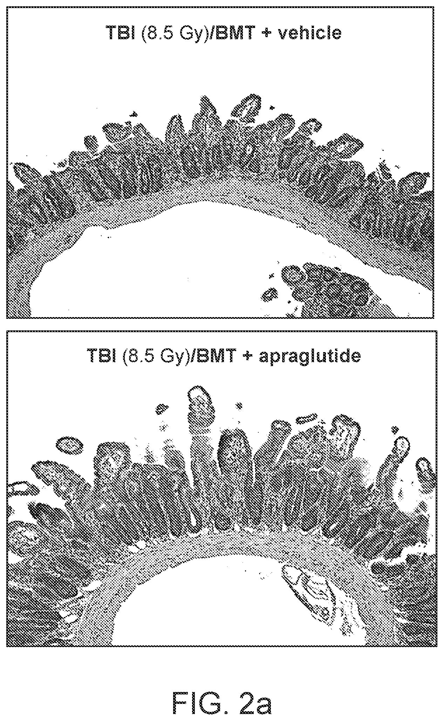

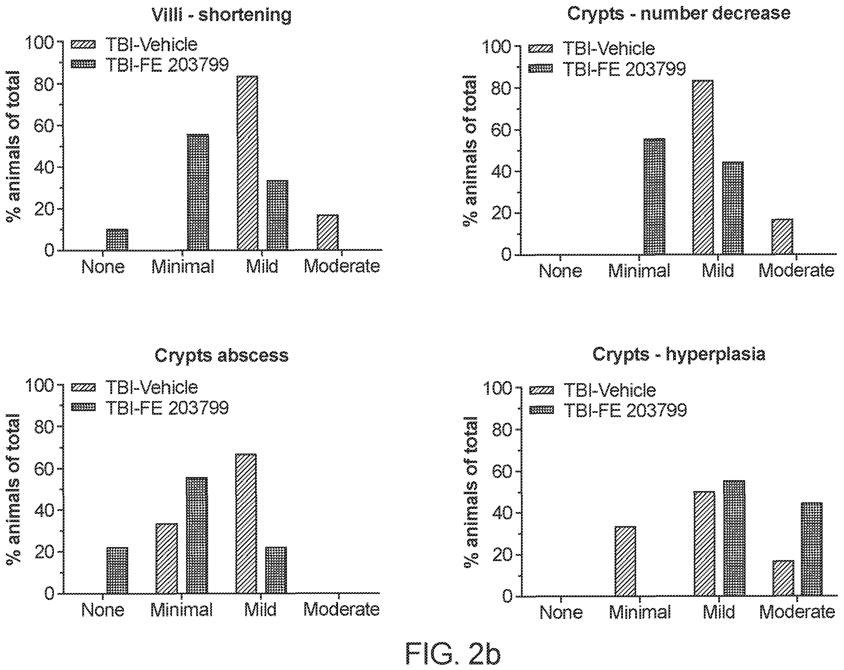

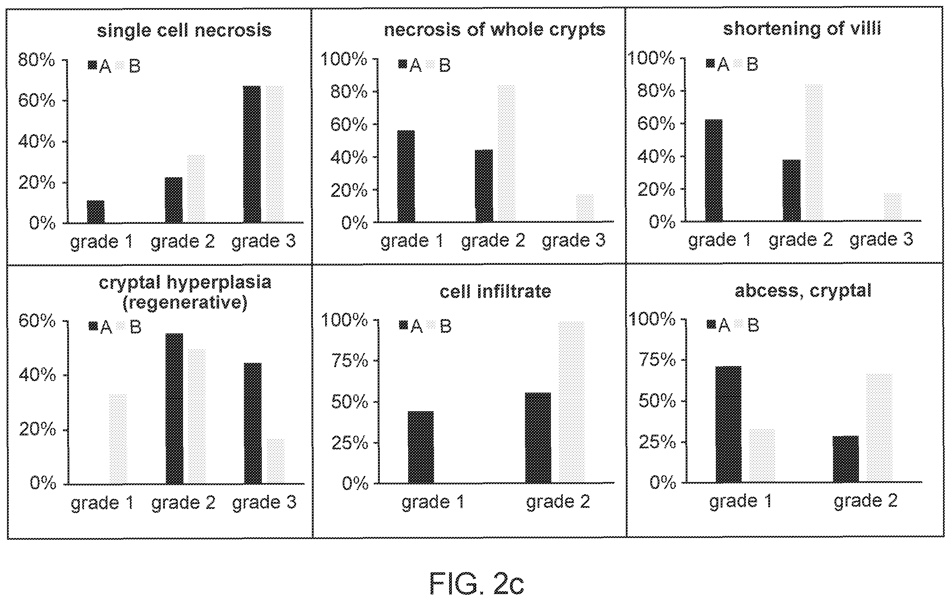

Various histological parameters of the jejunum were also measured, including single cell necrosis levels, necrosis of all crypts, shortening of villi, cryptal hyperplasia (regenerative), cell infiltrate, and cryptal abscess. These histological parameters were graded on a standard histological grading system (1 to 3). The results of the histological analysis are shown in FIG. 2a- 2c. In mice treated with apraglutide, the overall mean severity score of mucosal degenerative/inflammatory changes (shortening of villi [villous atrophy], mononuclear [lymphohistiocytic] /neutrophilic cell infiltrate in the lamina propria/intra-cryptal epithelium, decreased number of crypts and cryptal abscess) was slightly decreased in the jejunum, when compared with the animals treated with vehicle. Additionally, the mean severity score of the regenerative cryptal hyperplasia was slightly increased, when compared with the animals treated with vehicle. These indicate that the administration of apraglutide has a protective effective against jejunal mucosal damages induced by irradiation and GvHD.

In a second experiment BALB/cJ male mice were separated into three groups, Group A, Group B and Group C. Groups A and B consisted of 10 mice and Group C consisted of 5 mice.

Mice in Group A received subcutaneous injections of apraglutide in an amount of 3 mg/kg/mouse (administered in 300 µl of vehicle), every second day from Day -9 to Day +7 (Days: -9, -7, -5, -3 -1, +1, +3, +5, +7).

Mice in Group B received subcutaneous injections of 300 µl of vehicle control every second day starting from Day -9 to Day +7 (Days: -9, -7, -5, -3 -1, +1, +3, +5, +7).

Mice in Group C received subcutaneous injections of 300 µl of vehicle control every second day starting from Day -9 to Day +7 (Days: -9, -7, -5, -3 -1, +1, +3, +5, +7).

On Day -1 of the experiment, all mice in Groups A and B were irradiated with 7.0 Gy using an linac x-ray source. Group C was not irradiated but were injected with the vehicle only.

On Day 0 of the experiment, bone marrow and T cells from C57BL/6J mice were collected, prepared and transplanted into the mice of Group A and Group B. Each mouse in Group A and Group B was intravenously injected with 10 million bone marrows cells extracted from the femurs of the C57BL/6J mice and 2.5 million spleen cells extracted from the C57BL/6J mice.

Irradiated mice in Group A that were treated with apraglutide exhibited enhanced survival as compared to the irradiate, untreated mice in Group B. Group A had a median survival of 9 days and Group B had a median survival of 8.5 days. Moreover, 40% of apraglutide treated mice survived past Day 9 of the experiment. Group C mice received only the vehicle and were expected to survive. FIG. 14

Morbidity, mortality, body weight, clinical signs (diarrhea, appearance and behavior), length of intestinal segments, and histopathology of the intestine, skin, spleen and liver were monitored in the mice of Group A, Group B and Group C. All mice were sacrificed at Day +14 at the latest.

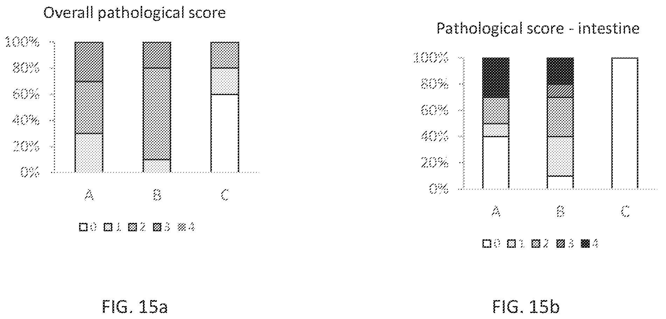

Diarrhea, appearance and behavior of the mice were coded into a pathological score (0, least severe to 4, most severe) (FIG. 15a). Macroscopically, four out of out of ten mice in group A showed an intestinal pathological score of 0 as compared to only a single mouse in group B (FIG. 15b).

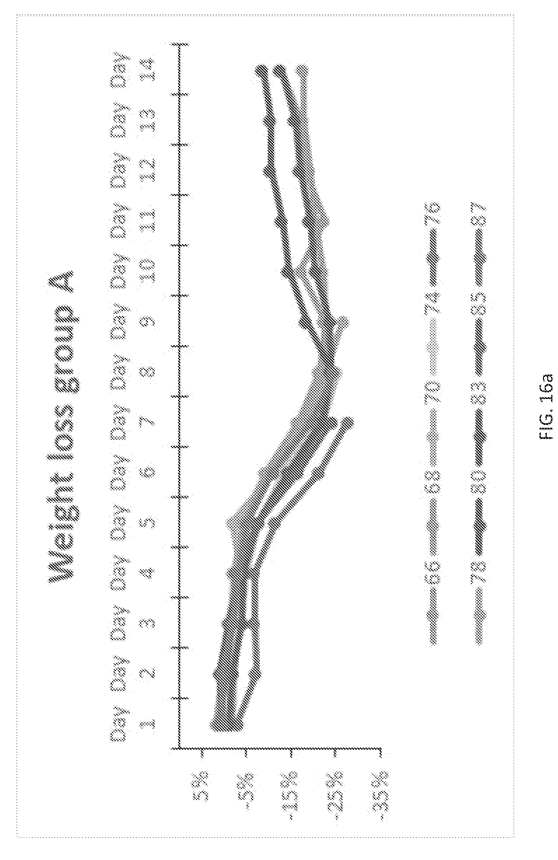

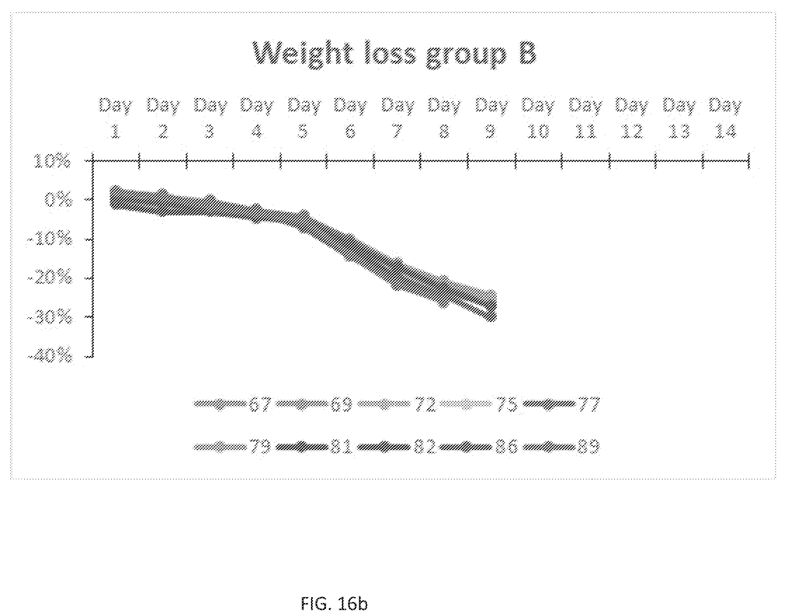

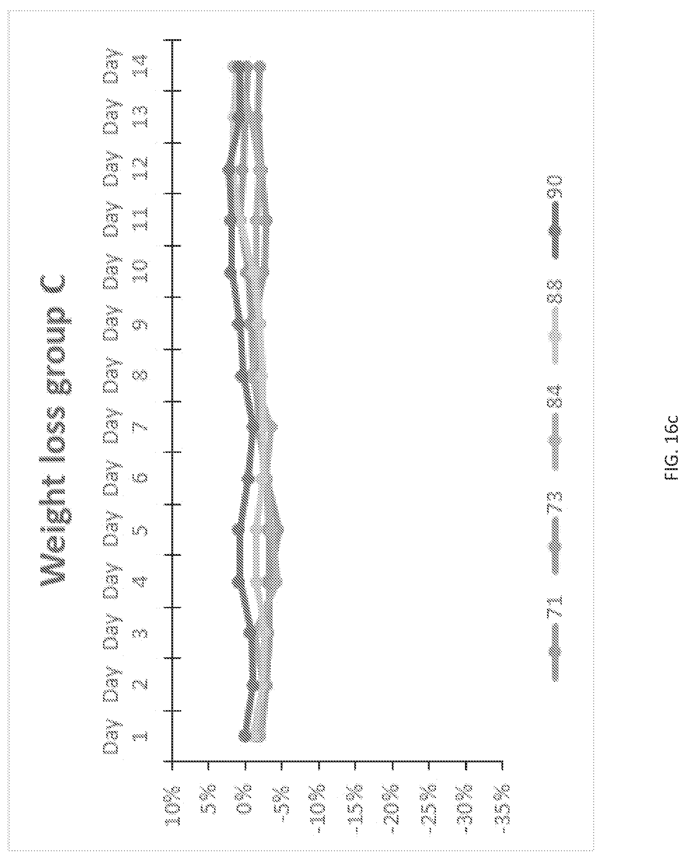

Weight loss was monitored in all three groups. No significant difference in weight loss was observed between Group A and Group B. In Group A the surviving mice beyond Day 9 regained body weight, while the mice in Group C did not gain weight (FIG. 16a-c).

Following sacrifice of the mice, the colon lengths and small intestine lengths of the mice in Group A, Group B and Group C were measured. The results of this analysis are shown in FIG. 3. As shown in FIG. 3, there was a clear difference in colon lengths in Group A as compared to Group B, as the mice that were treated with apraglutide showed increased colon lengths that were nearly the same as the mice in Group C. Thus, treatment with apraglutide protected the mice in Group A from a reduction in colon length, which is typically attributed to the inflammation caused by acute, gastrointestinal GvHDWithout wishing to be bound by theory, the results from the above-described experiments demonstrate that apraglutide can be used to treat acute gastrointestinal GvHD. As shown in FIG. 3, no differences were observed in the length of the small intestine in any of the three Groups. Without wishing to be bound by theory, the results from the above-described experiments demonstrate that apraglutide can be used to treat acute gastrointestinal GvHD. Specifically, apraglutide protects against gut lesions and reduction in colon lengths in the context of acute gastrointestinal GvHD induced by irradiation and allogeneic transplantation.

### Example 2

In a first experiment, total-body-irradiated (TBI) (1.44 Gy) immunodeficient (NOG) mice (Day 0) were injected with human peripheral blood mononuclear cell (hPBMC; 3x10⁷; Day 2) and treated with apraglutide 3.3 mg/kg or vehicle on Days -6 to 18). Engraftment rate was determined through CD45 expression in blood, bone marrow and spleen.

In a second experiment, TBI-induced intestinal damaged BALB/cJ mice received allogeneic transplantation and were treated with apraglutide (3.3 mg/kg) or vehicle on Days -9, - 7, -5, -3 -1, +1, +3, +5, +7. Intestinal damage indicative of GvHD (histological changes, length, hemorrhage, inflammation), body weight, and survival were assessed.

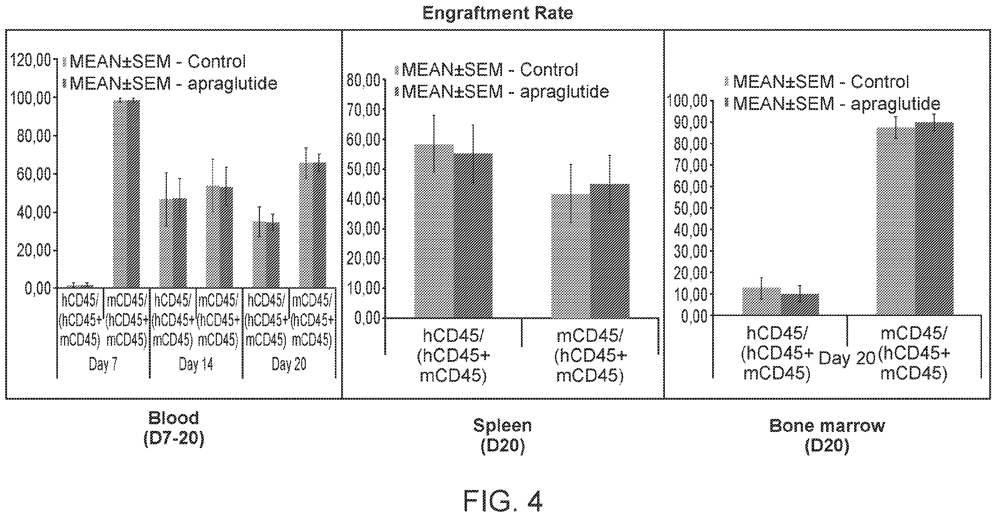

In the first experiment, hPBMC were successfully engrafted. Engraftment rate in blood, spleen and bone marrow was not affected by apraglutide (range 22.2-47.6% at D20 in blood). hCD45+ cells infiltration was observed in the intestinal wall with no difference between apraglutide vs vehicle (FIG. 4).

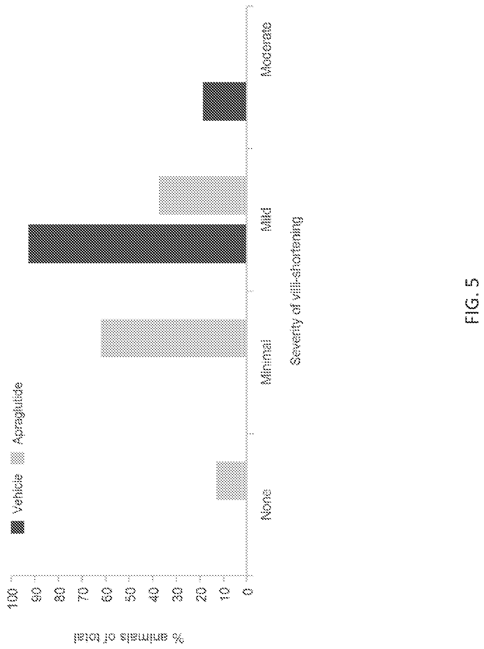

In the second experiment, the lymphocyte engraftment was successfully achieved in both apraglutide- and vehicle-treated mice. Weight loss and median survival were similar in both groups, but apraglutide-treated mice achieved significantly higher overall survival vs vehicle on Day +9 (40% vs 0%, respectively; p=0.0134). Post-mortem histological examination revealed less mucosal degenerative/inflammatory changes (villous atrophy, mononuclear/neutrophilic cell infiltrate in the lamina propria/intra-cryptal epithelium, crypt necrosis) in apraglutide-treated mice vs vehicle. Mean colon length in the apraglutide group (8.6±0.35 cm) was comparable to mice that did not undergo irradiation or transplantation (9.6±0.33 cm), whereas a significant reduction was apparent in the vehicle group (7.19±0.10 cm; p <0.05) (FIG. 5).

Without wishing to be bound by theory, the results from the experiments described above demonstrate that apraglutide treatment prior to allogeneic transplantation in immunodeficient mice did not affect the successful engraftment nor the rate of the engraftment. Furthermore, apraglutide showed a significant protective effect in TBI- and allogeneic-transplant-induced GvHD with reduced villi atrophy, minimize colon shortening, less severe intestinal damage, and showed a survival advantage. These findings support a beneficial role of apraglutide in reducing GI damage and limiting mortality from GvHD. Without wishing to be bound by theory, the results from the experiments described above supports that apraglutide decreases the severity of intestinal damage from acute gastrointestinal graft versus host disease (GI-GvHD) following allogeneic transplantation without impacting engraftment.

### Example 3

In a first experiment, 4 groups of Balb/c mice: (A) vehicle only; (B) cytarabine on Days 5-9, no apraglutide given; (C) cytarabine on Days 5-9; concomitant apraglutide on Days 5-18; (D) cytarabine on Days 5-9; pre-treatment apraglutide on Days 1, 3, and continued as concomitant treatment on Days 5, 8, 11, 14, and 17. Treatment groups were dosed with 30 mg/kg cytarabine and/or apraglutide 3.3 mg/kg.

In a second experiment, 3 treatment groups of Balb/c mice: (A) vehicle only; (B) melphalan on Day 9, no apraglutide; (C) melphalan on Day 9; pre-treatment apraglutide on Days 1, 3, 5, 7 and continued as concomitant treatment on Days 9, 11, and 13.

In both models, mice that received vehicle without any treatment served as controls. Intestinal tissue histology, body weight, survival, and plasma citrulline, a marker of total mucosal mass and intestinal growth, was assessed in both models.

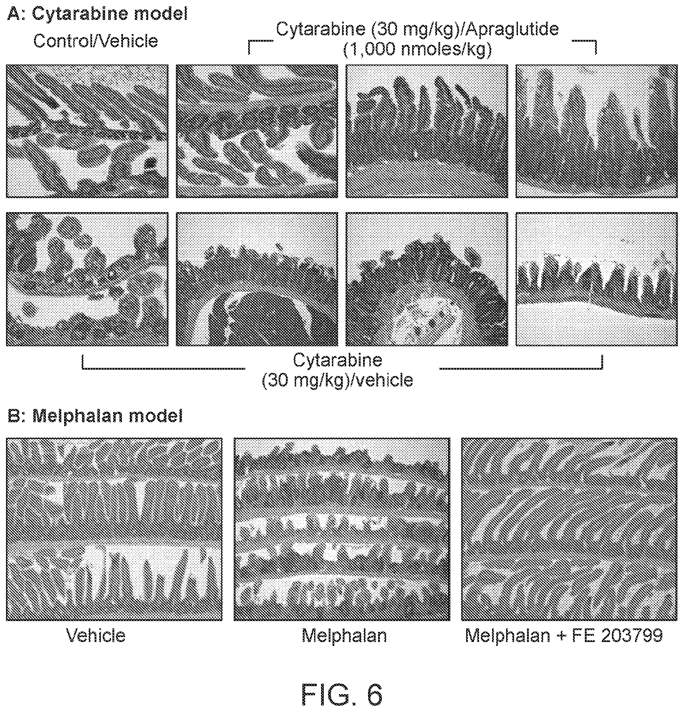

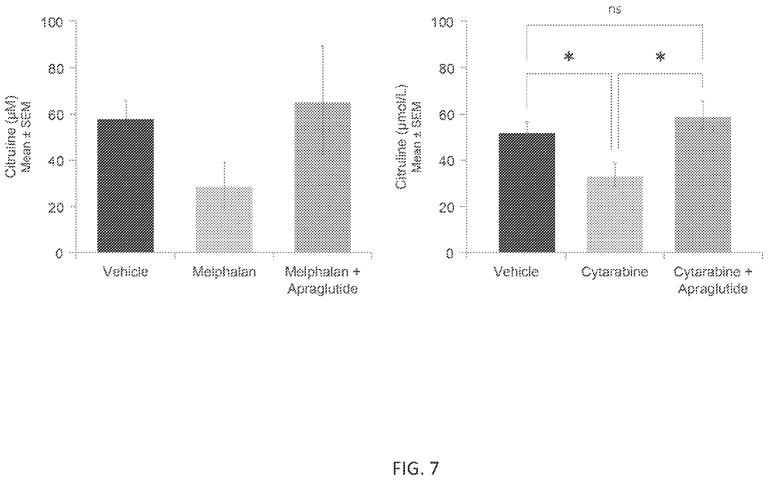

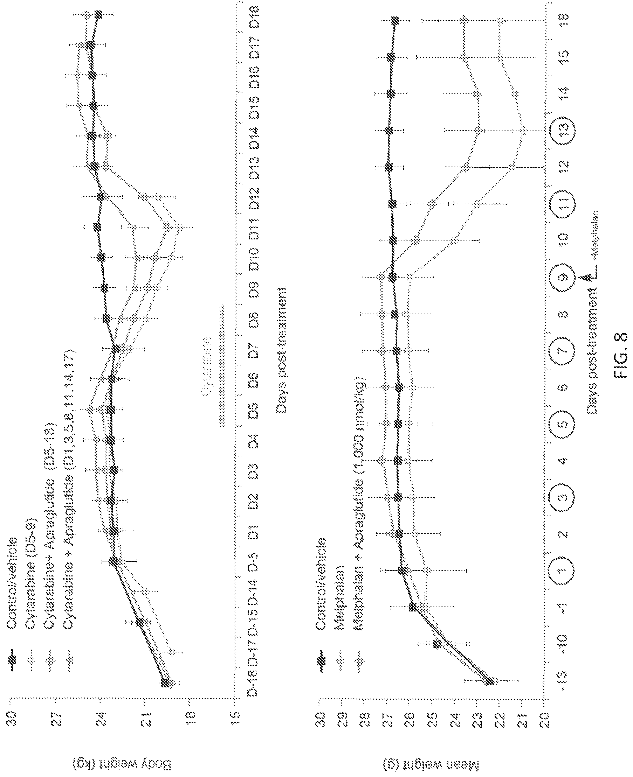

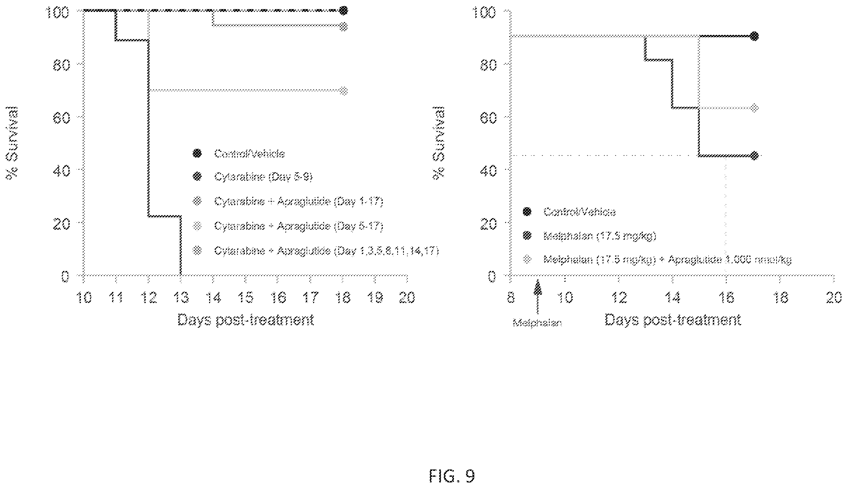

Histological examination showed that the degenerative intestinal changes (villi and crypt atrophy) caused by cytarabine or melphalan were reduced by apraglutide co-administration (FIG. 6). This was demonstrated by similarities in tissue morphology between vehicle-treated and mice treated with apraglutide. In addition, the duodenum, ileum and jejunum increased in weight with apraglutide. The intestinal protective effects of apraglutide were further supported by the preservation of plasma citrulline (indicative of intestinal mass) with apraglutide-treated mice having similar levels to that of vehicle-treated (FIG. 7). Apraglutide attenuated chemotherapy-induced weight loss (FIG. 8) and improved overall survival vs vehicle-only or chemotherapy-only groups (FIG. 9). The effects of apraglutide were optimal when it was administered as pre-treatment before chemotherapy.

Without wishing to be bound by theory, the results the microscopic examination showed apraglutide protected GI epithelium structure from chemotherapy-induced injury, improved survival, and prevented severe body weight loss in mice undergoing chemotherapy. Apraglutide also preserved intestinal mass as demonstrated by maintaining plasma citrulline level comparable to mice that did not undergo chemotherapy.

Without wishing to be bound by theory, the results from the experiments described above supports that apraglutide treatment reduces chemotherapy-induced gastrointestinal (GI) damage in mice and preserves the cellular integrity during chemotherapy.

### Example 4

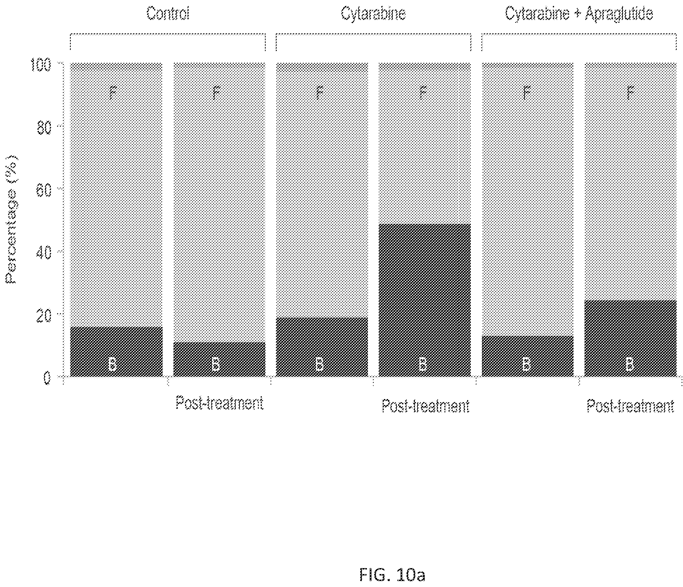

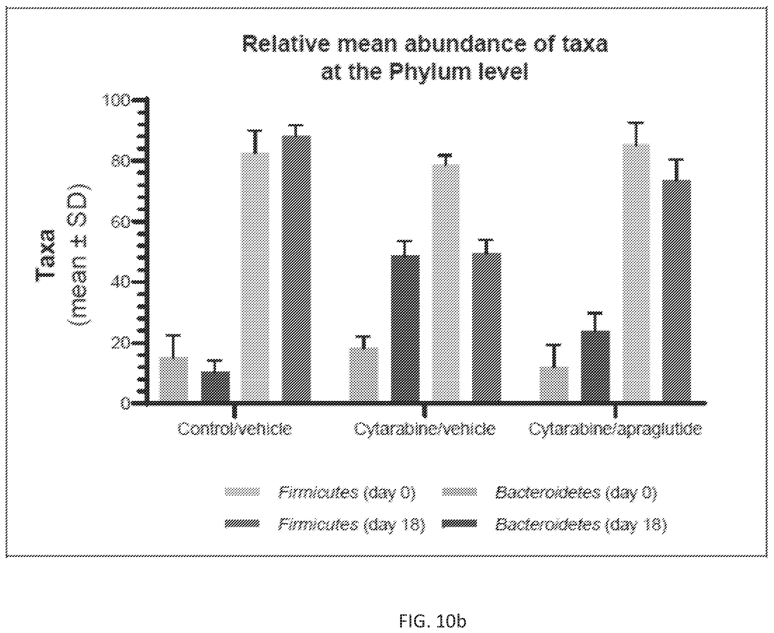

In a first experiment, Balb/c mice received cytarabine 30 mg/kg on Days 5-9 and apraglutide 3.3 mg/kg on Days 1-18. Control mice received vehicle on Days 1-18. Fecal samples were collected for bacterial phenotyping over ~24 hours at pre-treatment and the day before scheduled termination, and for found dead or pre-terminally euthanized animals. Microbiota composition were determined by 16S taxonomical meta-sequencing. Bacteroidetes and Firmicutes were the 2 main bacterial phyla identified (FIG. 10a and FIG. 10b). Chemotherapy with cytarabine caused significant changes in the composition of bacterial species, increasing the Bacteroidetes population and decreasing the proportion of Firmicutes bacteria. The change in levels of Bacteroidetes and Firmicutes bacteria from Days 0 to 18 was significantly greater in both the cytarabine-only and cytarabine + apraglutide mice vs vehicle (Table 1a and Table 1b). However, the effect was reduced by apraglutide co-administration. The difference in the change between cytarabine-only and cytarabine + apraglutide groups reached statistical significance for both Bacteroidetes (0.2486; p<0.0001) and Firmicutes (0.2037; p<0.0001). In addition, the ratio of Bacteroidetes to Firmicutes bacteria present remained more constant in cytarabine + apraglutide than in cytarabine-only group.

**Table 1a: Apraglutide treatment reduces chemotherapy-induced changes in fecal bacterial composition**

| | **Mean % of taxa at the Phylum level** | | | | | |
|---|---|---|---|---|---|---|
| | **Bacteroidetes** | | | **Firmicutes** | | |
| | **Day 0** | **Day 18** | **P-value for change Day 0 - Day 18** | **Day 0** | **Day 18** | **P-value for change Day 0 - Day 18** |
| **Group 1 (Vehicle only)** | 15.2 | 10.5 | 0.01723 | 82.6 | 88.2 | 0.1374 |
| **Group 2 (Cytarabine only)** | 16.3 | 48.7 | <0.0001 | 78.7 | 49.5 | <0.0001 |
| **Group 3 (Cytarabine + apraglutide)** | 12.2 | 24.0 | 0.0057 | 85.3 | 73.7 | 0.0107 |

| | | | | | | |
|---|---|---|---|---|---|---|
| *For each taxa, an analysis of variance for repeated measurements (repeated ANOVA) including treatment*, *day and treatment by day interaction as fixed factors in the statistical model was conducted. Post-hoc tests were conducted to perform pairwise comparisons of treatment groups on day 0 (pre-treatment) and on day 18 (post-treatment), using tukey adjustement for multiple testing.* | | | | | | |

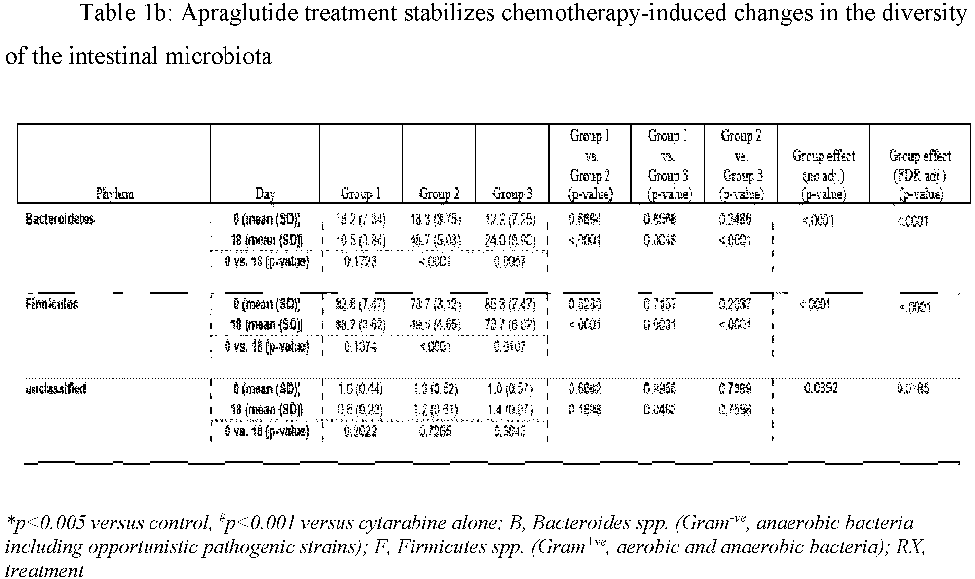

The treatment of mice with chemotherapy had a profound impact on bacterial homeostasis in the intestine, with a notable increase in opportunistic pathogenic bacteria populations. The proportions of different bacterial phyla in feces tended to remain closer to normal when apraglutide was co-administered with chemotherapy. Without wishing to be bound by theory, the results from the experiment described above demonstrate that the treatment with apraglutide resulted in preservation of the global homeostatic environment of the intestinal microbiota which is likely to contribute to the improved clinical outcomes (reduced body weight loss, increased survival) observed when apraglutide is administered concomitantly with chemotherapy agents. Moreover, as would be appreciated by the skilled artisan, imbalances in the gut microbiota are known to be a drive of GvHD pathogenesis (*see* Fredricks, J. Clin. Invest. 2019, 129(5): 1808-1817). Thus, apraglutide's ability to stabilize the intestinal microbiota indicates that it can be used to effectively treat and prevent GvHD.

### Example 5

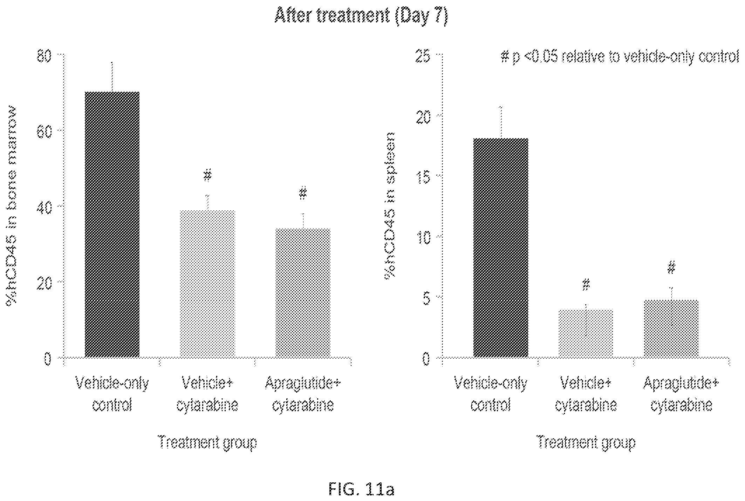

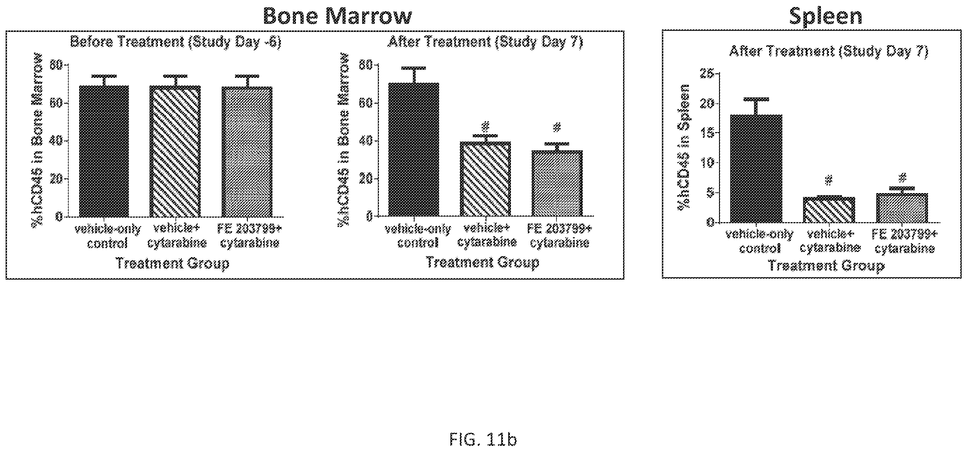

In a first experiment, cytarabine's anti-tumor effects were assessed in leukemic NOD/SCID mice (FIG. 11a). Apraglutide or vehicle was administered on Days -4 to 4. Cytarabine or vehicle was administered on Days 0-4. Bone marrow and spleen samples were collected on Day 7 and percentage of hCD45+ cells determined. There was no significant difference in % hCD45 cells detected in the bone marrow or spleen samples between the groups treated with cytarabine + vehicle and with cytarabine + apraglutide after treatment (FIG. 11b).

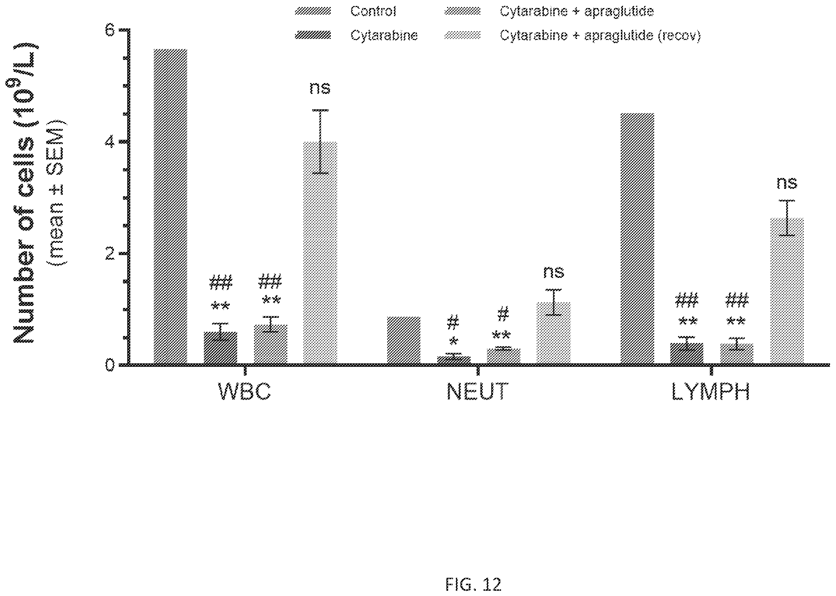

In a second experiment, apraglutide's effect on the ability of cytarabine to induce immunosuppression was assessed. The experiment included three groups of Balb/c mice: (A) vehicle; (B) cytarabine on Days 5-9; (C) cytarabine on Days 5-9, concomitant apraglutide on Days 5-18. RBC, platelets, WBC, NEU and LYMPH were assessed. A cohort was allowed to survive for 4 weeks to assess the effect of apraglutide on immunosuppression recovery (FIG. 12).

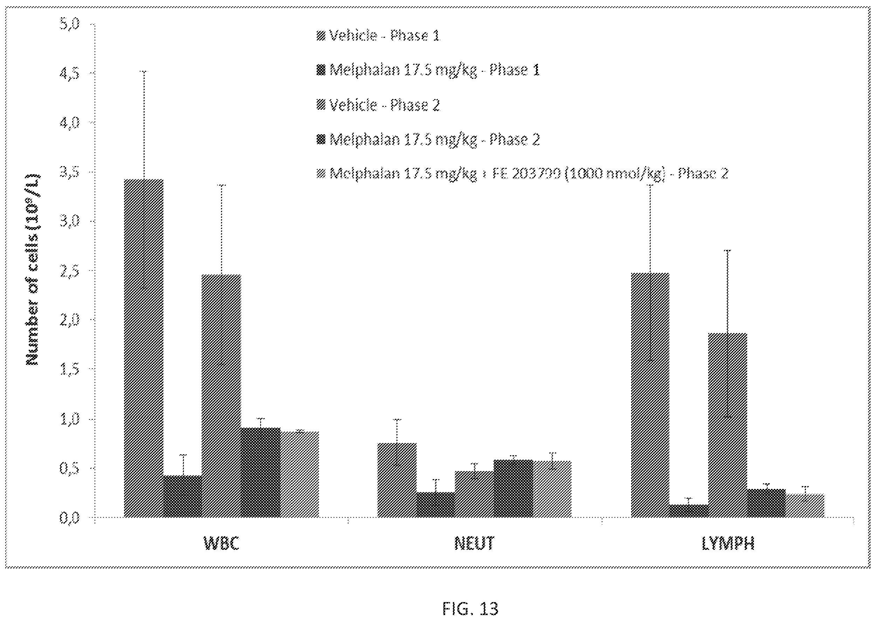

In a third experiment, the effect of apraglutide on melphalan-induced immunosuppression was assessed (FIG. 13). Three groups of Balb/c mice were included: (A) vehicle; (B) melphalan on Day 9; (C) melphalan on Day 9, apraglutide pre-treatment on Days 1, 3, 5, 7 and continued as co-administration on Days 9, 11, and 13. WBC, NEU and LYMPH were assessed.

In the first experiment, the reduction in human leukemia cells did not differ significantly between cytarabine-only and cytarabine + apraglutide, and was significantly greater than vehicle. The percentage of hCD45 in bone marrow after chemotherapy was 35.5±4 with cytarabine-only and 33.9±4.2 with cytarabine + apraglutide.

A dramatic decrease in leukocytes at the end of the treatment period in the second experiment, indicated that cytarabine-induced immunosuppression was not impaired by apraglutide co-administration (91% reduction in lymphocytes with both cytarabine + apraglutide and cytarabine-only) (Table 2). Apraglutide did not impact recovery of hematological parameters 4 weeks after the end of treatment.

**Table 2. Apraglutide does not affect cytarabine immunosuppression.**

| **Blood cell count** | **Percentage difference from control** | | |
|---|---|---|---|
| | **Cytarabine only** | **Cytarabine** + **Apraglutide 3.3 mg/kg^{a}** | **Cytarabine** + **Apraglutide 3.3 mg/kg^{a}** |
| | At treatment end | At treatment end | After 4-week recovery |
| **Red blood cells** | -23 | -8 | -5 |
| **Platelets** | -68 | 45 | 0 |
| **White blood cells** | -89 | -87 | -29 |
| **Lymphocytes** | -91 | -91 | -42 |
| **Neutrophils** | -82 | -64 | 30 |

| | | | |
|---|---|---|---|
| ^{a}Administered 4 days before start of cytarabine treatment until 9 days after end of cytarabine treatment | | | |

The third experiment showed that melphalan did elicit immunosuppression evidenced by leukocyte decrease. Mice treated with melphalan with or without apraglutide had severe decreases in WBC and LYMPH vs vehicle.

Without wishing to be bound by theory, the results from the experiments described above demonstrate that Pre- and concomitant apraglutide did not impair the efficacy of cytarabine in destroying human leukemia cells in vivo. Moreover, combination with apraglutide had no negative impact on cytarabine- or melphalan-induced immunosuppression. This pharmacologic effect of apraglutide was specific to the intestine and had no impact on the anti-tumor or immunosuppressive effects of cytarabine or melphalan. Apraglutide does not have an impact on anti-tumor and immunosuppressive efficacy of conditioning chemotherapy in mice.

### Example 6

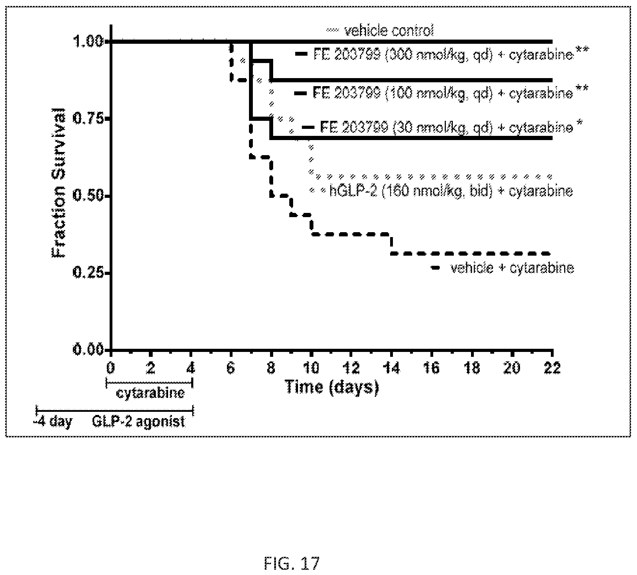

In a first experiment BALB/cJ female mice were separated into six groups for a dose-response study to assess the pharmacologic effect of increasing doses of apraglutide (0.11, 0.3 and 1.1 mg/kg) administered subcutaneously. Cytarabine was administered intraperitoneally at 30 mg/kg twice per day for 5 days (Day 0 to Day +4). Apraglutide was administered daily for a total of nine days, 4 days before and 5 days during the administration of chemotherapy (Day -4 to Day +4). Apraglutide effect was compared to unmodified hGLP-2 which was administered twice daily from Day -4 to Day +4, at a nominal dose of 0.60 mg/kg and combined with cytarabine. Results for animal survival showed a significant improvement of the animal survival as compared to cytarabine alone (FIG. 17).

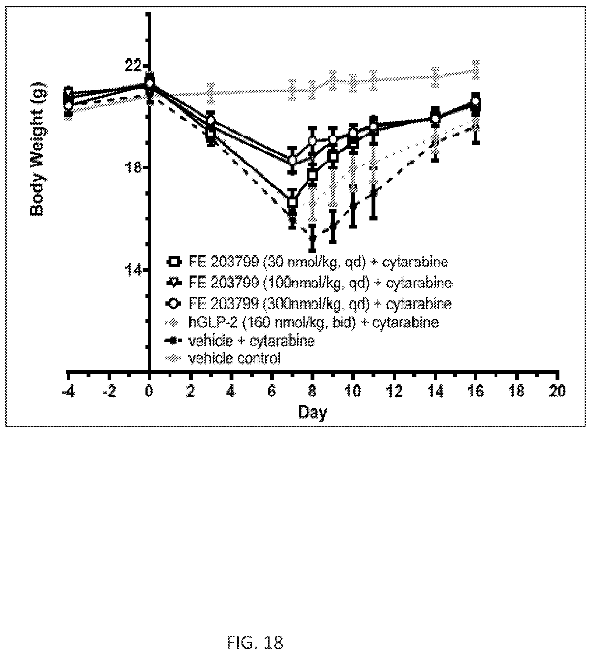

The animal survival with increasing doses of apraglutide was 11/16 at 0.11 mg/kg, 14/16 at 0.33 mg/kg and 16/16 at 1.1 mg/kg. The improvement in animal survival was statistically significant for all apraglutide-treated groups. While treatments with combination of cytarabine with 0.11 mg/kg apraglutide or hGLP-2 did not decrease initial weight loss, a trend towards increased body weight and survival was seen in such animals recovering from cytarabine treatment (FIG 18).

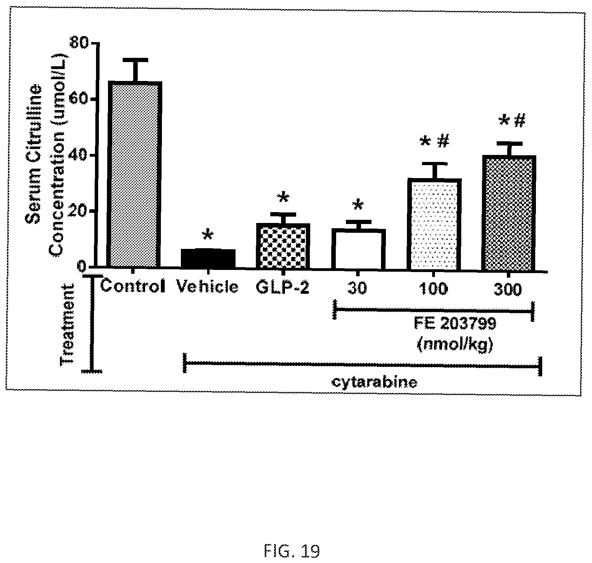

The intestinal protective effects of apraglutide were supported by the preservation of plasma citrulline (indicative of intestinal mass) with an improvement in citrulline concentrations when cytarabine was co-administered with apraglutide (0.33 mg/kg and 1.1 mg/kg) compared to cytarabine alone-treated animals (FIG. 19).

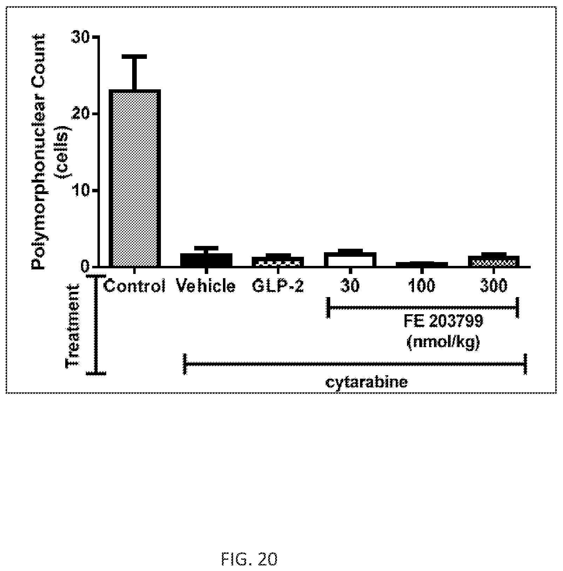

The apraglutide dose-dependent impact on the immunosuppressive effect of cytarabine was evaluated. The effect of cytarabine treatment on polymorphonuclear (PMN) cell count was determined (FIG. 20). Cytarabine alone-treated animals showed depleted PMN counts which were not reversed by hGLP-2 or apraglutide combined therapy suggesting that improvement in animal survival and body weight was not a result of improved neutrophil granulocyte count, but rather maintenance of GI integrity. In a separate study during which animals treated with cytarabine alone or in combination with apraglutide initiated before cytarabine (Days-4 to +12) were allowed to survive for 4 additional weeks.

### Example 7

In a first experiment the safety and efficacy of apraglutide in subjects with Grade II to IV (MAGIC) steroid refractory gastrointestinal (GI) acute graft versus host disease on best available therapy will be evaluated in a randomized, double-blind trial.

Inclusion Criteria for randomized, double-blind Phase 2 study of apraglutide:
1. Participants are aged 12 years or above at the time of consent and who weigh a minimum of 40 kg;
2. Participants undergoing alloSCT from any donor source (matched unrelated donor, sibling, haplo-identical) using bone marrow, peripheral blood stem cells, or cord blood. Recipients of non-myeloablative, myeloablative, and reduced intensity conditioning are eligible;
3. Participants have evident myeloid and platelet engraftment (confirmed prior to trial medication start): a) Absolute neutrophil count >1000/mm3; and b) Platelets ≥20,000/mm3;
4. Participants use of growth factor supplementation (granulocyte-colony stimulating factor and granulocyte-macrophage-colony stimulating factor) and transfusion support is allowed;
5. Participants have histologically diagnosed GI-aGVHD at screening (with clinically confirmed SR GI-aGVHD at ruxolitinib start and prior to apraglutide start) defined as subjects administered SS, given alone or combined with calcineurin inhibitors (CNI) and either:
   a) Disease progression based on organ assessment after 3 days of systemic methylprednisolone (MP) of 2mg/kg +/- CNI; or
   b) Did not improve after 7 days of treatment with MP 2mg/kg/day equivalent; or
   c) Progressed to a new organ after treatment with MP 2 mg/kg/day equivalent for skin and upper GI-aGVHD; or
   d) Recurred during or after a steroid taper.
   All subjects must have Stage 1-4 lower GI-aGVHD at enrollment.
6. Participants may be treated with systemic steroids (SS) plus ruxolitinib (RUX) (RUX at the recommended dose 2 twice daily for 0-3 days). Calcineurin inhibitors are allowed as co-medication, if needed.

Participants weighing ≥50.0 kg, will be randomized to one of two treatment arms (low dose or high dose within three body weight bands). The exact dose they receive at each visit will depend upon the weight band they fall into (Table 3). Participants who weigh 40.0-49.9 kg will receive apraglutide 2.5 mg. Apraglutide will be administered subcutaneously, once weekly for between 8-13 weeks. If the subjects will benefit from continuing to receive apraglutide, they can receive it for a maximum of 26 weeks.

**Table 3: Dose of Investigational Medicinal Product Based on Treatment Arm and Weight**

| **Treatment Arm** | **Weight Band (based on Baseline Weight)** | | | |
|---|---|---|---|---|
| | **40.0-<50.0 kg** | **50.0-<60.0 kg** | **60.0-80.0 kg** | **>80.0 kg** |
| Low dose (mg) | 2.5 | 2.5 | 4 | 5 |
| High dose (mg) | | 5 | 7.5 | 10 |

Initially, participants undergo screening assessments during a 14-day Screening Period and provide informed consent. Screening assessments include, but are not limited to, analysis of analysis of vital signs, height and weight, colonoscopy, gut biopsy, medical history, and the like.

This study will be a randomized, double-blind, repeat dose, trial to evaluate the safety and efficacy of apraglutide in subjects with Grade II to IV SR GI-aGVHD who are taking BAT. Thirty-four subjects, who have been taking RUX for 0-3 days on a background of SS, will receive a dose of apraglutide SC once weekly for either 8 weeks or 13 weeks. In addition to the N=30 subjects randomized to receive low dose or high dose apraglutide SC once weekly for 8 or 13 weeks (based on three body weight bands), a separate, non-randomized cohort of up to four subjects with a body weight 40.0-49.9 kg will be assigned to receive apraglutide 2.5 mg. All subjects complete a safety follow-up visit at week 17 (120 (± 7) days) after the first dose of study drug. Safety and efficacy follow-up assessments will continue to be performed for 2 years after the first dose of apraglutide (Week 104).

The primary objective is to assess safety and tolerability of apraglutide in subjects with SR GI-aGVHD of the mid-lower GI tract who are treated with SS and with RUX for 0-3 days. The primary efficacy endpoint is to assess gastrointestinal-aGVHD response at Day 56 in subjects with GI-aGVHD treated with apraglutide, SS, and RUX compared with SS and RUX alone.

The foregoing discussion discloses and describes merely exemplary embodiments of this disclosure.

## Claims

1. Apraglutide, or a pharmaceutically acceptable salt thereof, for use in the treatment or prevention of acute gastrointestinal Graft versus Host Disease (GvHD) in a subject.

2. The apraglutide or pharmaceutically acceptable salt thereof for use of claim 1, wherein the acute gastrointestinal GvHD is steroid-refractory.

3. The apraglutide or pharmaceutically acceptable salt thereof for use of claim 1, wherein the acute gastrointestinal GvHD is steroid-naive.

4. The apraglutide or pharmaceutically acceptable salt thereof for use of any one of claims 1-3, wherein the acute gastrointestinal GvHD is grade II- IV acute gastrointestinal GvHD according to the MAGIC scale.

5. The apraglutide or pharmaceutically acceptable salt thereof for use of any one of claims 1-4, wherein the apraglutide or pharmaceutically acceptable salt thereof is administered to the subject:
(a) prior to the subject being administered a transplant; or
(b) prior the subject being administered radiation therapy, chemotherapy, or radiomimetic therapy in connection with a transplant.

6. The apraglutide or pharmaceutically acceptable salt thereof for use of any one of claims 1-5, wherein:
(a) the subject has been previously administered a transplant, preferably wherein the apraglutide or pharmaceutically acceptable salt thereof is administered to the subject after the subject has been administered a transplant; and/or
(b) the subject has been previously administered radiation therapy, chemotherapy, radiomimetic therapy or any combination thereof in connection with a transplant, preferably wherein the apraglutide or pharmaceutically acceptable salt thereof is administered to the subject after the subject has been administered radiation therapy, chemotherapy, radiomimetic therapy or any combination thereof.

7. The apraglutide or pharmaceutically acceptable salt thereof for use of any one of claims 1-6, wherein the apraglutide or pharmaceutically acceptable salt thereof is administered to the subject after the subject has been administered radiation therapy, chemotherapy, radiomimetic therapy or any combination thereof and prior to the subject being administered a transplant.

8. The apraglutide or pharmaceutically acceptable salt thereof for use of any one of claims 1-7, wherein the apraglutide or pharmaceutically acceptable salt thereof is administered to the subject:
(a) concurrently with a transplant; or
(b) concurrently with a radiation therapy, a chemotherapy, a radiomimetic therapy, or any combination thereof in connection with a transplant.

9. The apraglutide or pharmaceutically acceptable salt thereof for use of any one of claims 1-8, wherein the administration of apraglutide prevents and/or attenuates a reduction in colon length in a subject following:
(a) a transplant; and/or
(b) a conditioning therapy and a transplant.

10. The apraglutide or pharmaceutically acceptable salt thereof for use of any one of claims 1-9, wherein:
(a) the transplant comprises hematopoietic stem cells derived from bone marrow, peripheral blood, umbilical cord blood or any combination thereof, preferably wherein the transplant comprises T-cells, preferably wherein the T-cells are chimeric antigen receptor (CAR) T-cells; or
(b) the radiation therapy comprises total body irradiation.

11. The apraglutide or pharmaceutically acceptable salt thereof for use of any one of claims 1-10, wherein the pharmaceutically acceptable salt of apraglutide is the sodium salt of apraglutide.

12. The apraglutide or pharmaceutically acceptable salt thereof for use of any one of claims 1-11, wherein the apraglutide, or pharmaceutically acceptable salt thereof, is administered by subcutaneous injection.

13. The apraglutide or pharmaceutically acceptable salt thereof for use of any one of claims 1-12, wherein the apraglutide, or pharmaceutically acceptable salt thereof, is administered in an amount of between 1 mg to 10 mg, optionally wherein
(a) the apraglutide is administered in an amount of about 2.5 mg; or
(b) the apraglutide is administered in an amount of about 5 mg; or
(c) the apraglutide is administered in an amount of about 10 mg.

14. The apraglutide or pharmaceutically acceptable salt thereof for use of any one of claims 1-13, wherein
(a) the subject has a body weight of less than about 50 kg and the apraglutide is administered in an amount of about 2.5 mg;
(b) the subject has a body weight of about 50 kg to about 60 kg and the apraglutide is administered in an amount of about 2.5 mg or about 5 mg;
(c) the subject has a body weight of about 60 kg to about 80 kg and the apraglutide is administered in an amount of about 4 mg or about 7.5 mg; or
(d) the subject has a body weight of greater than about 80 kg and the apraglutide is administered in an amount of about 5 mg or about 10 mg.

15. The apraglutide or pharmaceutically acceptable salt thereof for use of any one of claims 5-14, wherein the transplant is:
(a) an allogeneic transplant; or
(b) autologous transplant.

16. The apraglutide or pharmaceutically acceptable salt thereof for use of any one of claims 1-15, wherein the subject has been previously administered at least one anti-GvHD therapy, preferably wherein the at least one anti-GvHD therapy comprises steroid therapy.

## Patentansprüche

1. Apraglutid oder pharmazeutisch unbedenkliches Salz davon zur Verwendung bei der Behandlung oder Vorbeugung von akuter gastrointestinaler Graft-versus-Host-Krankheit (Graft versus Host Disease, GvHD) bei einem Individuum.

2. Apraglutid oder pharmazeutisch unbedenkliches Salz davon zur Verwendung nach Anspruch 1, wobei die akute gastrointestinale GvHD steroidrefraktär ist.

3. Apraglutid oder pharmazeutisch unbedenkliches Salz davon zur Verwendung nach Anspruch 1, wobei die akute gastrointestinale GvHD steroidnaiv ist.

4. Apraglutid oder pharmazeutisch unbedenkliches Salz davon zur Verwendung nach einem der Ansprüche 1-3, wobei es sich bei der akuten gastrointestinalen GvHD um akute gastrointestinale GvHD Grad II-IV gemäß der MAGIC-Skala handelt.

5. Apraglutid oder pharmazeutisch unbedenkliches Salz davon zur Verwendung nach einem der Ansprüche 1-4, wobei das Apraglutid oder pharmazeutisch unbedenkliche Salz davon dem Individuum verabreicht wird:
(a) bevor dem Individuum eine Transplantation verabreicht wird; oder
(b) bevor dem Individuum eine Strahlentherapie, Chemotherapie oder radiomimetische Therapie in Verbindung mit einer Transplantation verabreicht wird.

6. Apraglutid oder pharmazeutisch unbedenkliches Salz davon zur Verwendung nach einem der Ansprüche 1-5, wobei:
(a) dem Individuum zuvor eine Transplantation verabreicht wurde, vorzugsweise wobei das Apraglutid oder pharmazeutisch unbedenkliche Salz davon dem Individuum verabreicht wird, nachdem dem Individuum eine Transplantation verabreicht wurde; und/oder
(b) dem Individuum zuvor eine Strahlentherapie, Chemotherapie, radiomimetische Therapie oder irgendeine Kombination davon in Verbindung mit einer Transplantation verabreicht wurde, vorzugsweise wobei das Apraglutid oder pharmazeutisch unbedenkliche Salz davon dem Individuum verabreicht wird, nachdem dem Individuum eine Strahlentherapie, Chemotherapie, radiomimetische Therapie oder eine beliebige Kombination davon verabreicht wurde.

7. Apraglutid oder pharmazeutisch unbedenkliches Salz davon zur Verwendung nach einem der Ansprüche 1-6, wobei das Apraglutid oder pharmazeutisch unbedenkliche Salz davon dem Individuum verabreicht wird, nachdem dem Individuum eine Strahlentherapie, Chemotherapie, radiomimetische Therapie oder irgendeine Kombination davon verabreicht wurde und bevor dem Individuum eine Transplantation verabreicht wird.

8. Apraglutid oder pharmazeutisch unbedenkliches Salz davon zur Verwendung nach einem der Ansprüche 1-7, wobei das Apraglutid oder pharmazeutisch unbedenkliche Salz davon dem Individuum verabreicht wird:
(a) gleichzeitig mit einer Transplantation; oder
(b) gleichzeitig mit einer Strahlentherapie, einer Chemotherapie, einer radiomimetischen Therapie oder irgendeiner Kombination davon in Verbindung mit einer Transplantation.

9. Apraglutid oder pharmazeutisch unbedenkliches Salz davon zur Verwendung nach einem der Ansprüche 1-8, wobei die Verabreichung von Apraglutid eine Verringerung der Kolonlänge bei einem Individuum verhindert und/oder abschwächt nach:
(a) einer Transplantation; und/oder
(b) einer Konditionierungstherapie und einer Transplantation.

10. Apraglutid oder pharmazeutisch unbedenkliches Salz davon zur Verwendung nach einem der Ansprüche 1-9, wobei:
(a) das Transplantat hämatopoetische Stammzellen umfasst, die aus Knochenmark, peripherem Blut, Nabelschnurblut oder irgendeiner Kombination davon stammen, vorzugsweise wobei das Transplantat T-Zellen umfasst, vorzugsweise wobei es sich bei den T-Zellen um Chimärer-Antigenrezeptor(CAR)-T-Zellen handelt; oder
(b) die Strahlentherapie eine Ganzkörperbestrahlung umfasst.

11. Apraglutid oder pharmazeutisch unbedenkliches Salz davon zur Verwendung nach einem der Ansprüche 1-10, wobei es sich bei dem pharmazeutisch unbedenklichen Salz von Apraglutid um das Natriumsalz von Apraglutid handelt.

12. Apraglutid oder pharmazeutisch unbedenkliches Salz davon zur Verwendung nach einem der Ansprüche 1-11, wobei das Apraglutid oder pharmazeutisch unbedenkliche Salz davon durch subkutane Injektion verabreicht wird.

13. Apraglutid oder pharmazeutisch unbedenkliches Salz davon zur Verwendung nach einem der Ansprüche 1-12, wobei das Apraglutid oder pharmazeutisch unbedenkliche Salz davon in einer Menge zwischen 1 mg und 10 mg verabreicht wird, gegebenenfalls wobei
(a) das Apraglutid in einer Menge von etwa 2,5 mg verabreicht wird; oder
(b) das Apraglutid in einer Menge von etwa 5 mg verabreicht wird; oder
(c) das Apraglutid in einer Menge von etwa 10 mg verabreicht wird.

14. Apraglutid oder pharmazeutisch unbedenkliches Salz davon zur Verwendung nach einem der Ansprüche 1-13, wobei
(a) das Individuum ein Körpergewicht von weniger als etwa 50 kg aufweist und das Apraglutid in einer Menge von etwa 2,5 mg verabreicht wird;
(b) das Individuum ein Körpergewicht von etwa 50 kg bis etwa 60 kg aufweist und das Apraglutid in einer Menge von etwa 2,5 mg oder etwa 5 mg verabreicht wird;
(c) das Individuum ein Körpergewicht von etwa 60 kg bis etwa 80 kg aufweist und das Apraglutid in einer Menge von etwa 4 mg oder etwa 7,5 mg verabreicht wird; oder
(d) das Individuum ein Körpergewicht von mehr als etwa 80 kg aufweist und das Apraglutid in einer Menge von etwa 5 mg oder etwa 10 mg verabreicht wird.

15. Apraglutid oder pharmazeutisch unbedenkliches Salz davon zur Verwendung nach einem der Ansprüche 5-14, wobei es sich bei der Transplantation um:
(a) eine allogene Transplantation oder
(b) autologe Transplantation handelt.

16. Apraglutid oder pharmazeutisch unbedenkliches Salz davon zur Verwendung nach einem der Ansprüche 1-15, wobei dem Individuum zuvor mindestens eine Anti-GvHD-Therapie verabreicht wurde, vorzugsweise wobei die mindestens eine Anti-GvHD-Therapie eine Steroidtherapie umfasst.

## Revendications

1. Apraglutide, ou sel pharmaceutiquement acceptable de celui-ci, pour utilisation dans le traitement ou la prévention d'une maladie gastro-intestinale du greffon contre l'hôte (GvHD) aiguë chez un sujet.

2. Apraglutide ou sel pharmaceutiquement acceptable de celui-ci pour utilisation selon la revendication 1, dans lequel la GvHD gastro-intestinale aiguë est réfractaire aux stéroïdes.

3. Apraglutide ou sel pharmaceutiquement acceptable de celui-ci pour utilisation selon la revendication 1, dans lequel la GvHD gastro-intestinale aiguë est naïve de stéroïdes.

4. Apraglutide ou sel pharmaceutiquement acceptable de celui-ci pour utilisation selon l'une quelconque des revendications 1 à 3, dans lequel la GvHD gastro-intestinale aiguë est la GvHD gastro-intestinale aiguë de grade II-IV selon l'échelle MAGIC.

5. Apraglutide ou sel pharmaceutiquement acceptable de celui-ci pour utilisation selon l'une quelconque des revendications 1 à 4, dans lequel l'apraglutide ou un sel pharmaceutiquement acceptable de celui-ci est administré au sujet :
(a) avant que le sujet ne reçoive une transplantation ; ou
(b) avant que le sujet ne reçoive une radiothérapie, une chimiothérapie ou une thérapie radiomimétique en connexion avec une transplantation.

6. Apraglutide ou sel pharmaceutiquement acceptable de celui-ci pour utilisation selon l'une quelconque des revendications 1 à 5, dans lequel :
(a) le sujet a précédemment reçu une transplantation, de préférence dans lequel l'apraglutide ou le sel pharmaceutiquement acceptable de celui-ci est administré au sujet après que le sujet a reçu une transplantation ; et/ou
(b) le sujet a déjà reçu une radiothérapie, une chimiothérapie, une thérapie radiomimétique ou une quelconque combinaison de celles-ci en connexion avec une transplantation, de préférence dans lequel l'apraglutide ou le sel pharmaceutiquement acceptable de celui-ci est administré au sujet après que le sujet a reçu une radiothérapie, une chimiothérapie, une thérapie radiomimétique ou une quelconque combinaison de celles-ci.

7. Apraglutide ou sel pharmaceutiquement acceptable de celui-ci pour utilisation selon l'une quelconque des revendications 1 à 6, dans lequel l'apraglutide ou le sel pharmaceutiquement acceptable de celui-ci est administré au sujet après que le sujet a reçu une radiothérapie, une chimiothérapie, une thérapie radiomimétique ou une quelconque combinaison de celles-ci et avant que le sujet ne reçoive une transplantation.

8. Apraglutide ou sel pharmaceutiquement acceptable de celui-ci pour utilisation selon l'une quelconque des revendications 1 à 7, dans lequel l'apraglutide ou un sel pharmaceutiquement acceptable de celui-ci est administré au sujet :
(a) simultanément avec une transplantation ; ou
(b) simultanément avec une radiothérapie, une chimiothérapie, une thérapie radiomimétique ou une quelconque combinaison de celles-ci en connexion avec une transplantation.

9. Apraglutide ou sel pharmaceutiquement acceptable de celui-ci pour utilisation selon l'une quelconque des revendications 1 à 8, dans lequel l'administration d'apraglutide empêche et/ou atténue une réduction de la longueur du côlon chez un sujet après :
(a) une transplantation ; et/ou
(b) une thérapie de conditionnement et une transplantation.

10. Apraglutide ou sel pharmaceutiquement acceptable de celui-ci pour utilisation selon l'une quelconque des revendications 1 à 9, dans lequel :
(a) la transplantation comprend des cellules souches hématopoïétiques dérivées de la moelle osseuse, du sang périphérique, du sang du cordon ombilical ou d'une quelconque combinaison de ceux-ci, de préférence dans lequel la transplantation comprend des cellules T, de préférence dans lequel les cellules T sont des cellules T de récepteur antigénique chimérique (CAR) ; ou
(b) la radiothérapie comprend une irradiation corporelle totale.

11. Apraglutide ou sel pharmaceutiquement acceptable de celui-ci pour utilisation selon l'une quelconque des revendications 1 à 10, dans lequel le sel pharmaceutiquement acceptable d'apraglutide est le sel de sodium d'apraglutide.

12. Apraglutide ou sel pharmaceutiquement acceptable de celui-ci pour utilisation selon l'une quelconque des revendications 1 à 11, dans lequel l'apraglutide, ou un sel pharmaceutiquement acceptable de celui-ci, est administré par injection sous-cutanée.

13. Apraglutide ou sel pharmaceutiquement acceptable de celui-ci pour utilisation selon l'une quelconque des revendications 1 à 12, dans lequel l'apraglutide, ou un sel pharmaceutiquement acceptable de celui-ci, est administré en une quantité comprise entre 1 mg et 10 mg, éventuellement dans lequel
(a) l'apraglutide est administré en une quantité d'environ 2,5 mg ; ou
(b) l'apraglutide est administré en une quantité d'environ 5 mg ; ou
(c) l'apraglutide est administré en une quantité d'environ 10 mg.

14. Apraglutide ou sel pharmaceutiquement acceptable de celui-ci pour utilisation selon l'une quelconque des revendications 1 à 13, dans lequel
(a) le sujet a un poids corporel inférieur à environ 50 kg et l'apraglutide est administré en une quantité d'environ 2,5 mg ;
(b) le sujet a un poids corporel d'environ 50 kg à environ 60 kg et l'apraglutide est administré en une quantité d'environ 2,5 mg ou d'environ 5 mg ;
(c) le sujet a un poids corporel d'environ 60 kg à environ 80 kg et l'apraglutide est administré en une quantité d'environ 4 mg ou d'environ 7,5 mg ; ou
(d) le sujet a un poids corporel supérieur à environ 80 kg et l'apraglutide est administré en une quantité d'environ 5 mg ou d'environ 10 mg.

15. Apraglutide ou sel pharmaceutiquement acceptable de celui-ci pour utilisation selon l'une quelconque des revendications 5 à 14, dans lequel la transplantation est :
(a) une transplantation allogénique ; ou
(b) une transplantation autologue.

16. Apraglutide ou sel pharmaceutiquement acceptable de celui-ci pour utilisation selon l'une quelconque des revendications 1 à 15, dans lequel le sujet a reçu précédemment au moins une thérapie anti-GvHD, de préférence dans lequel l'au moins une thérapie anti-GvHD comprend une thérapie stéroïdienne.
